# EUROPEAN PATENT SPECIFICATION

(11) **EP 4 382 049 B1**
(45) Date of publication and mention of the grant of the patent: **22.10.2025**
(21) Application number: 23214534.2
(22) Date of filing: 06.12.2023
(51) Int. Cl.: A61B 6/03, A61B 6/10, A61B 6/12

(54) **MEDICAL SYSTEM AND RECORDING MEDIA**
MEDIZINISCHES SYSTEM UND AUFZEICHNUNGSMEDIUM
SYSTÈME MÉDICAL ET SUPPORT D'ENREGISTREMENT

(30) Priority: 06.12.2022 JP 2022195239
(43) Date of publication of application: 12.06.2024
(73) Proprietor: GE Precision Healthcare LLC, Waukesha, WI 53188 (US)
(72) Inventor: NUKUI, Masatake, Hino 191-8503 (JP); ISHIHARA, Yotaro, Hino 191-8503 (JP); TERAOKA, Yuri, Hino 191-8503 (JP)
(74) Representative: Kilburn & Strode LLP

(56) References cited:
- US-A1- 2017 220 716
- US-A1- 2020 391 054
- US-A1- 2022 296 188

## Description

### [TECHNICAL FIELD]

The present invention relates to a medical system for displaying scattered radiation distribution, and to a recording medium on which an instruction for controlling the medical system is recorded.

### [BACKGROUND ART]

A CT device is known as a medical device that noninvasively images a subject body. CT devices can capture images of an imaging site in a short period of time, and therefore have become widespread in hospitals and other medical facilities.

On the other hand, CT devices use X-rays to examine patients, and as CT devices become more widespread, there is increasing concern about patient exposure during examinations. Thus, it is important to control patient exposure dose from the perspective of reducing the patient exposure dose from X-rays as much as possible and the like. Therefore, technologies to control the dose have been developed. For example, Patent Document 1 discloses a dose control system.

US2017/220716 is directed to a method for estimating a spatial distribution of the hazardousness of radiation doses for individuals evolving in a medical operating room defining a three-dimensional environment surrounding at least one source of radiation.

US2022/296188 is directed to a mobile X-ray device that includes a system controller for actuating the X-ray device, a calculation unit for real-time determination of a scattered radiation distribution from an X-ray radiation generated by the X-ray source in at least parts of the surrounding area of the X-ray device, and a display unit that is arranged on the device trolley and/or the recording system. The display unit is configured for display, taking place substantially in real time, of at least one item of information dependent upon the determined scattered radiation distribution in at least one part of the surroundings of the mobile X-ray device.

US2020/391054 is directed to systems and methods for providing a real time visualization of scattered radiation in an operating room. A number of visualization devices such as augmented reality ("AR") tracking devices, electronic displays, or projection devices are in electronic communication with a controller and configured to display a visualization of scattered radiation.

### [CITATION LIST]

### [PATENT DOCUMENT]

Patent Document 1: Japanese Unexamined Patent Application Publication 2015-173889

### SUMMARY

An invention is set out in the claims.

Furthermore, in recent years, CT-guided puncture, in which CT images are used as a guide to puncture a patient, has also been utilized. In CT-guided puncture, the surgeon can perform puncture while acquiring CT images of the patient, and therefore can accurately puncture a target (tumor and the like) within the patient. Therefore, CT-guided puncture is an effective method for biopsy, cancer treatment, and the like.

On the other hand, the surgeon is exposed to X-rays irradiated from an X-ray tube during CT-guided puncture. Therefore, surgeons wear protective clothing and use shields when necessary to reduce exposure. Furthermore, as of April 2021, the maximum exposure limit for the lens of the eye of radiology personnel and the like has been lowered to 50 mSv/year. This has also increased the importance of wearing protective eyewear. Furthermore, needle guides are also used to reduce the surgeon's exposure.

However, X-rays irradiated from an X-ray tube are repeatedly scattered inside the patient's body and also inside the gantry of the CT device, and are irradiated outside the patient's body and the CT device as scattered radiation. Although needle guides are very effective for exposure to direct radiation, exposure to scattered radiation cannot be ignored even when a needle guide is used. Therefore, it is important to reduce exposure to scattered radiation.

However, unlike direct radiation, scattered radiation has the characteristic of traveling in various directions, and therefore, the surgeon cannot visually determine the level of scattered radiation intensity at the surgeon's own standing position. Therefore, the surgeon cannot recognize the relationship between the current standing position and the scattered radiation intensity, making it difficult to take measures against exposure to the scattered radiation.

Furthermore, during CT-guided puncture, not only the surgeon but also the subject body is exposed to scattered radiation. Therefore, it is important to reduce not only the exposure of the surgeon but also the exposure of the subject body. However, as described above, unlike direct radiation, scattered radiation has the characteristic of traveling in various directions, making it difficult to visually determine the level of the scattered radiation intensity at the position of the subject body undergoing treatment.

Therefore, it is desirable to have a technology that allows a surgeon and/or subject body to visually recognize the level of exposure to scattered radiation.

A first example disclosed herein is a medical system, including:
a gantry including an X-ray tube;
a table; and
one or more processors, wherein
the one or more processors execute operations including:
   identifying the position and shape of a surgeon and/or subject body in a scan room where the gantry and table are installed;
   identifying, based on the position and shape of the surgeon and/or subject body, a first scattered radiation distribution in a region of the scan room in which the surgeon and/or subject body is present from a three-dimensional scattered radiation distribution that three-dimensionally expresses the distribution of scattered radiation generated by scanning the subject body; and displaying the first scattered radiation distribution.

Furthermore, a second example disclosed herein is a method for displaying a scattered radiation distribution, the method including:
identifying the position and shape of a surgeon and/or subject body in a scan room where a gantry including an X-ray tube and table are installed;
identifying, based on the position and shape of the surgeon and/or subject body, a first scattered radiation distribution in a region of the scan room in which the surgeon and/or subject body is present from a three-dimensional scattered radiation distribution that three-dimensionally expresses the distribution of scattered radiation generated by scanning the subject body; and
displaying the first scattered radiation distribution.

Furthermore, a third example disclosed herein is one or more non-transitory, computer-readable recording media storing one or more instructions executable by one or more processors provided in a medical system including a gantry including an X-ray tube and a table, wherein the one or more instructions cause the one or more processors to execute an operation including:
identifying the position and shape of a surgeon and/or subject body in a scan room where the gantry and table are installed;
identifying, based on the position and shape of the surgeon and/or subject body, a first scattered radiation distribution in a region of the scan room in which the surgeon and/or subject body is present from a three-dimensional scattered radiation distribution that three-dimensionally expresses the distribution of scattered radiation generated by scanning the subject body; and
displaying the first scattered radiation distribution.

The present examples can identify a first scattered radiation distribution in a region where a surgeon and/or subject body is present in a scan room from three-dimensional scattered radiation distributions and display the first scattered radiation distribution. Therefore, the surgeon and/or subject body can be made visually aware of the level of exposure of the surgeon and/or subject body to scattered radiation.

### [BRIEF DESCRIPTION OF DRAWINGS]

FIG. 1 is a perspective view of a gantry 102 and table 116;
FIG. 2 is a block diagram of a CT system 100;
FIG. 3 is a front surface view of the gantry 102;
FIG. 4 is an explanatory diagram of an operation of an X-ray tube during measurement of a three-dimensional scattered radiation distribution;
FIG. 5 is a diagram schematically depicting a three-dimensional scattered radiation distribution A0;
FIG. 6 is a diagram schematically depicting a three-dimensional scattered radiation distribution 250;
FIG. 7 is an explanatory diagram of a half scan when a start angle and end angle of an X-ray tube 1104 are shifted by a prescribed angle α in clockwise direction;
FIG. 8 is an explanatory diagram of a half scan when the X-ray tube 1104 irradiates X-rays while rotating between a start angle of 330° and an end angle of 210°;
FIG. 9 is a diagram schematically depicting three-dimensional scattered radiation distributions A0 to A330 when a half scan is performed in accordance with a scan condition X1;
FIG. 10 is a diagram depicting three-dimensional scattered radiation distributions obtained for a plurality of scan conditions X1 to Xm;
FIG. 11 is a flowchart of CT-guided puncture;
FIG. 12 is a diagram depicting a surgeon 101 during the CT-guided puncture;
FIG. 13 is an explanatory diagram of a first half scan;
FIG. 14 is an explanatory diagram of step ST8;
FIG. 15 is an explanatory diagram of step ST9;
FIG. 16 is an explanatory diagram of step ST10;
FIG. 17 is an enlarged view of the surgeon 101 on which a scattered radiation distribution 11 is projected;
FIG. 18 is a diagram depicting a projection position of the scattered radiation distribution 11 when the surgeon 101 changes the standing position;
FIG. 19 is a diagram schematically depicting CT images 31, 32, and 33 displayed on an intervention monitor 237;
FIG. 20 is a diagram depicting the angle of an X-ray tube 104 at a point in time when a trigger T for starting a second half scan occurs;
FIG. 21 is an explanatory diagram of step ST85;
FIG. 22 is an explanatory diagram of step ST9;
FIG. 23 is an explanatory diagram of step ST10;
FIG. 24 is an enlarged view of the surgeon 101 on which a scattered radiation distribution 12 is projected;
FIG. 25 is an explanatory diagram of a cumulative scattered radiation distribution;
FIG. 26 is a diagram schematically depicting a cumulative scattered radiation distribution 14 expressing cumulative exposure of the scattered radiation on the surgeon 101 between the start and end of the CT-guided puncture;
FIG. 27 is an explanatory diagram of step ST85;
FIG. 28 is an explanatory diagram of step ST85;
FIG. 29 is a diagram depicting an operation flow of a CT system in Embodiment 2;
FIG. 30 is a flowchart of step ST22;
FIG. 31 is an explanatory diagram of a method for selecting a three-dimensional scattered radiation distribution that minimizes exposure of the surgeon 101;
FIG. 32 is an explanatory diagram of a first half scan;
FIG. 33 is a diagram depicting the surgeon 101 on which is projected a scattered radiation distribution 403 of a three-dimensional scattered radiation distribution A60 that minimizes exposure;
FIG. 34 is an explanatory diagram of step ST222 and step ST223; and
FIG. 35 is a diagram depicting an example in which a display device displays a scattered radiation distribution.

### [DESCRIPTION OF EMBODIMENTS]

An embodiment for carrying out the invention will be described below, but the present invention is not limited to the following embodiment.

### Embodiment 1

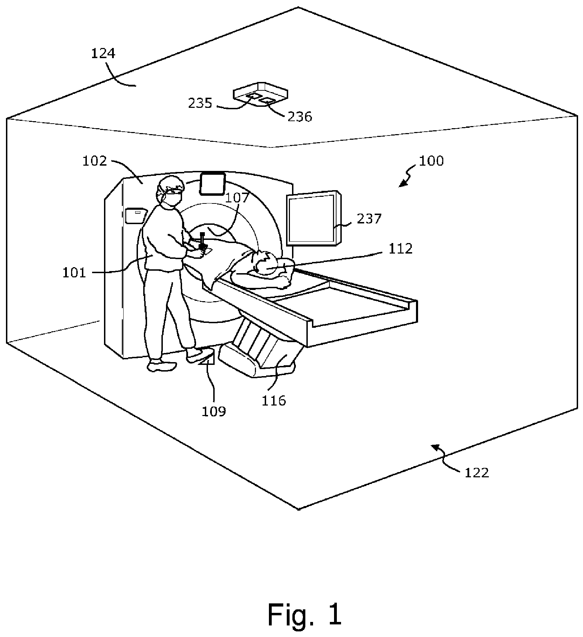

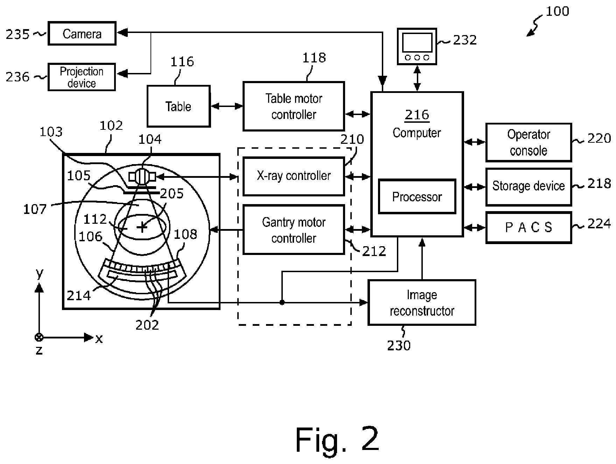

FIG. 1 is a perspective view of a gantry 102 and table 116 of a CT system 100 of Embodiment 1, and FIG. 2 is a block diagram of the CT system 100.

The CT system 100 includes a gantry 102 and a table 116. The gantry 102 and the table 116 are installed in a scan room 122.

The gantry 102 has an opening 107 and a subject body 112 is transported through the opening 107 to scan the subject body 112.

The gantry 102 is equipped with an X-ray tube 104, a filter part 103, a front collimator 105, an X-ray detector 108, and the like.

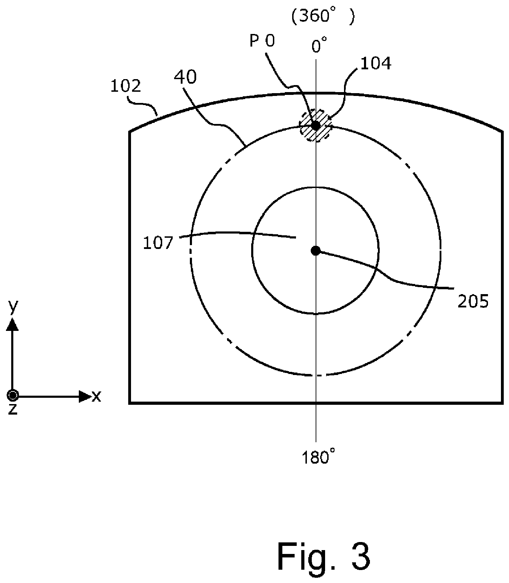

FIG. 3 is a front surface view of the gantry 102.
FIG. 3 depicts the X-ray tube 104 provided in the gantry 102.
The X-ray tube 104 generates X-rays when a prescribed voltage is applied to the cathode-anode tube. The X-ray tube 104 is configured so as to be rotatable on a path 40 along a circumference of a circle centered on a rotation axis 205 on the XY plane. Herein, the Z direction represents the body axis direction, the Y direction represents the vertical direction (the height direction of the table 116), and the X direction represents the direction perpendicular to the Z and Y directions. Note that an X-ray tube compatible with a rapid kV switching system capable of switching the tube voltage may be provided as the X-ray tube 104. Furthermore, in Embodiment 1, the CT system 100 includes one X-ray tube 104, but two X-ray tubes may be included.

In FIG. 3, the position of the X-ray tube 104 on the path 40 is represented by an angle. Specifically, a position P0 on the path 40 where a Y coordinate is maximum is defined as an angle of 0°, and the position of the X-ray tube 104 on the path 40 is defined as an angle of 0° to 360°. In FIG. 3, the X-ray tube 104 is depicted positioned at an angle of 0°. Note that the rotation axis 205 may be set so as to coincide with the isocenter, or may be set as a rotation axis 1205 at a position deviated from the isocenter.

Returning to FIG. 2, the description is continued.
The filter part 103 includes, for example, a flat plate filter and/or a bow-tie filter.
The front collimator 105 is a component that narrows the X-ray irradiation range such that X-rays are not irradiated in unwanted regions.

The X-ray detector 108 includes a plurality of detector elements 202. A plurality of detector elements 202 detect an X-ray beam 106 that is irradiated from the X-ray tube 104 and passes through the subject body 112, such as a patient or the like. Therefore, the X-ray detector 108 can acquire projection data for each view.

The projection data detected by the X-ray detector 108 is collected by a DAS 214. The DAS 214 performs prescribed processing, including sampling, digital conversion, and the like, on the collected projection data. The processed projection data is transmitted to a computer 216. The computer 216 stores the data from the DAS 214 in a storage device 218. The storage device 218 includes one or more recording media that store programs as well as instructions and the like to be executed by the processor. The recording medium can be, for example, one or more non-transitory, computer-readable recording media. The storage device 218 may include, for example, hard disk drives, floppy disk drives, compact disc read/write (CD-R/W) drives, digital versatile disk (DVD) drives, flash drives, and/or solid state storage drives.

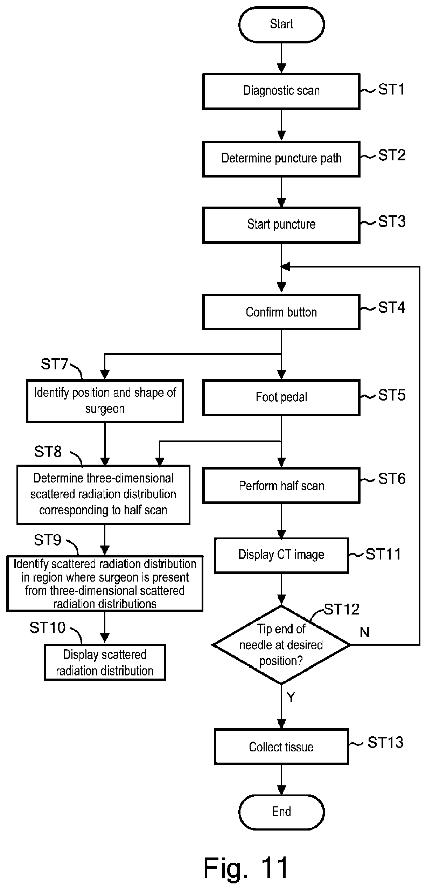

The computer 216 includes one or more processors. The processor of computer 216 is configured to be able to communicate with the storage device 218 (and/or external storage devices). The computer 216 uses one or more processors to output commands and parameters to the DAS 214, X-ray controller 210, and/or gantry motor controller 212, to control system operations such as data acquisition and/or processing and the like. Furthermore, the computer 216 uses one or more processors to execute various processes, such as signal processing, data processing, image processing, and the like, in each step of the flow described later (see FIG. 11, FIG. 14, FIG. 28, and FIG. 30).

An operator console 220 is linked to the computer 216. An operator can enter prescribed operator inputs related to the operation of the CT system 100 into the computer 216 by operating the operator console 220. The computer 216 receives operator input, including commands and/or scan parameters, via the operator console 220 and controls system operation based on that operator input. The operator console 220 can include a keyboard (not depicted) or touch screen for the operator to specify commands and/or scan parameters.

The X-ray controller 210 controls the X-ray tube 104 based on an instruction from the computer 216. Furthermore, the gantry motor controller 212 also controls a gantry motor to rotate a structural element, such as the X-ray tube 104, X-ray detector 108, and the like, based on instruction from the computer 216.

Furthermore, the CT system 100 includes a foot pedal 109 (see FIG. 1). In the Embodiment 1, the foot pedal 109 is provided to perform a half scan to acquire a CT image necessary for CT-guided puncture described later. Half scan will be described later.

FIG. 2 illustrates only one operator console 220, but two or more operator consoles may be linked to the computer 216.

The CT system 100 may also allow a plurality of remotely located displays, printers, workstations, and/or similar devices to be linked via, for example, wired and/or wireless networks.

In one embodiment, for example, the CT system 100 may include or be linked to a Picture Archiving and Communication System (PACS) 224. In a typical implementation, a PACS 224 may be linked to a remote system such as a radiology department information system, hospital information system, and/or internal or external network (not depicted) or the like.

The computer 216 provides instructions to a table motor controller 118 to control the table 116. The table motor controller 118 can control the table motor so as to move the table 116 based on the instructions received. For example, the table motor controller 118 can move the table 116 such that the subject body 112 is positioned appropriately for imaging.

As mentioned above, the DAS 214 samples and digitally converts the projection data acquired by the detector elements 202. The image reconstructor 230 then reconstructs the image using the sampled and digitally converted data. The image reconstructor 230 includes one or more processors, which can perform an image reconstruction process. In FIG. 2, the image reconstructor 230 is illustrated as a separate structural element from the computer 216, but the image reconstructor 230 may form a part of the computer 216. In addition, the computer 216 may also perform one or more functions of the image reconstructor 230. Furthermore, the image reconstructor 230 may be positioned away from the CT system 100 and operatively connected to the CT system 100 using a wired or wireless network.

The image reconstructor 230 can store the reconstructed image in the storage device 218. The image reconstructor 230 may also transmit the reconstructed image to the computer 216. The computer 216 can transmit the reconstructed image and/or patient information to a display device 232 communicatively linked to the computer 216 and/or image reconstructor 230.

The various methods and processes described in the present specification can be recorded as executable instructions on a non-transitory recording medium within the CT system 100. The executable instructions may be stored on a single recording medium or distributed across a plurality of recording media. One or more processors provided in the CT system 100 execute the various methods, steps, and processes described in the present specifications in accordance with the instructions recorded on a recording medium.

Furthermore, a camera 235 is provided on a ceiling 124 of the scan room 122 as an optical image acquisition unit for acquiring an optical image in the scan room. The processor of computer 216 can communicate with the camera 2325. Any optical image acquisition unit can be used as the optical image acquisition unit so long a subject such as a subject body or the like can be imaged. For example, a camera that uses visible light for imaging the subject, a camera that uses infrared for imaging the subject, or a depth sensor that uses infrared to acquire depth data of the subject and performs imaging of the surface of the subject based on the depth data, can be used as the optical image acquisition unit. Furthermore, the optical image acquired by the optical image acquisition unit may be a 3D image or a 2D image. Furthermore, the optical image acquisition unit may acquire the optical image as a still image or as video. The processor of the computer 216 identifies the position and shape of the surgeon 101 and/or subject body 112 based on the optical image.

Furthermore, the ceiling 124 of the scan room 122 is provided with a projection device 236 for projecting the scattered radiation distribution. The projection device 236 will be described later.

The CT system 100 is configured as described above. The CT system 100 of Embodiment 1 is configured so as to be able to perform CT-guided puncture. In the CT-guided puncture, the surgeon 101 (see FIG. 1) can perform puncture while acquiring CT images of the subject body, and therefore can accurately puncture a target (tumor and the like) within the subject body. Therefore, CT-guided puncture is an effective method for biopsy, cancer treatment, and the like.

On the other hand, the surgeon 101 is exposed to X-rays irradiated from the X-ray tube during CT-guided puncture. Therefore, the surgeon 101 wears protective clothing or uses a shield when necessary to reduce exposure. A needle guide or the like is used to reduce the exposure of the surgeon 101.

However, X-rays irradiated from an X-ray tube are repeatedly scattered inside the patient's body and also inside the gantry of the CT device, and are irradiated outside the patient's body and the CT device as scattered radiation. Although needle guides are very effective for exposure to direct radiation, exposure to scattered radiation cannot be ignored even when a needle guide is used. Therefore, it is important to reduce exposure to scattered radiation.

However, unlike direct radiation, scattered radiation has the characteristic of traveling in various directions, and therefore, the surgeon 101 cannot visually determine the level of scattered radiation intensity at the surgeon's own standing position. Therefore, the surgeon 101 cannot recognize the relationship between the current standing position and the scattered radiation intensity, making it difficult to take measures against exposure to the scattered radiation.

Therefore, in Embodiment 1, the CT system 100 operates such that the surgeon 101 can visually recognize the level of exposure of the surgeon 101 to scattered radiation at the standing position. Hereinafter, a description is given on how the CT system 100 allows the surgeon 101 to be visually aware of exposure to scattered radiation.

Note that in Embodiment 1, in order to make the surgeon 101 visually aware of exposure to scattered radiation, the three-dimensional scattered radiation distribution, which three-dimensionally expresses the distribution of scattered radiation generated by scanning the subject body, is measured and stored in the storage device 218 before performing CT-guided puncture on the subject body 112. Therefore, hereinafter, a method for measuring three-dimensional scattered radiation distribution will be described, followed by the flow of CT-guided puncture.

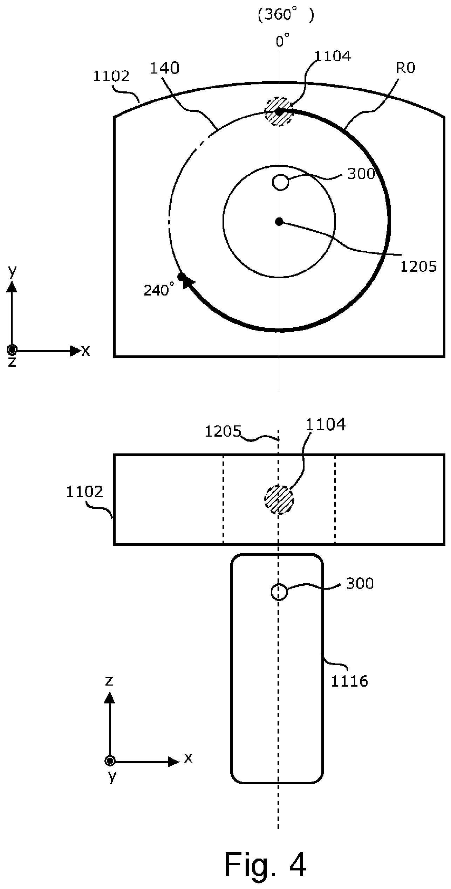

FIGS. 4 to 10 are explanatory diagrams depicting the measurement method for three-dimensional scattered radiation distribution.
In the present embodiment, a gantry 1102 and table 1116 of the same structure as the gantry 102 and table 116 of the CT system 100 (see FIG. 1) are used to measure the three-dimensional scattered radiation distribution. Hereinafter, a method for measuring the three-dimensional scattered radiation distribution using the gantry 1102 and table 1116 will be described.

FIG. 4 is an explanatory diagram of an operation of an X-ray tube during measurement of a three-dimensional scattered radiation distribution.
A front surface view of the gantry 1102 is depicted on the upper side of FIG. 4, and an upper surface view of the gantry 1102 and the table 1116 is depicted on the lower side of FIG. 4.

The gantry 1102 includes an X-ray tube 1104. The X-ray tube 1104 is configured so as to be rotatable on a path 140 along a circumference of a circle centered on a rotation axis 1205 on the XY plane. Furthermore, a dosimeter 300 for measuring scattered radiation is installed on an upper part of the table 1116.

In FIG. 4, the position of the X-ray tube 1104 on the path 140 is represented by an angle. Specifically, a position on the path 140 where a Y coordinate is maximum is defined as an angle of 0°, and the position of the X-ray tube 1104 on the path 140 is defined as an angle of 0° to 360°. In FIG. 4, the X-ray tube 1104 is depicted positioned at an angle of 0°. Note that the rotation axis 1205 may be set so as to coincide with the isocenter, or may be set as a rotation axis 1205 at a position deviated from the isocenter.

In Embodiment 1, measurement of the three-dimensional scattered radiation distribution when a half scan is performed shall be considered.

A half scan is a scan in which the X-ray tube 1104 is rotated by 180° + fan angle to collect projection data for a rotation angle of (180° + fan angle). For example, if the fan angle is 60°, a half scan is a scan in which projection data is collected for a rotation angle of 240°.

When performing a half scan, the angle of the X-ray tube at the start of the scan (hereinafter referred to as "start angle") and the angle of the X-ray tube at the end of the scan (hereinafter referred to as "end angle") are determined. In FIG. 4, the start angle of the X-ray tube 1104 is determined to be 0° and the end angle of the X-ray tube 1104 is determined to be 240°.

After determining the start angle and end angle of the X-ray tube 1104, the scattered radiation generated by the half scan are measured. When measuring the scattered radiation, first, the X-ray tube 1104 is rotated and time is allowed until the X-ray tube 1104 reaches the desired rotational speed. Once the rotational speed of the X-ray tube 1104 begins to stabilize, the X-ray tube 1104 is allowed to start irradiating X-rays at a point in time when the X-ray tube 1104 reaches the start angle of 0°. Furthermore, when the X-ray tube 1104 reaches the end angle of 240°, the X-ray tube 1104 finishes irradiating X-rays. Therefore, a half scan is performed by rotating the X-ray tube 1104 through an angle range R0 between the start angle of 0° and the end angle of 240° while irradiating X-rays. The dosimeter 300 measures the scattered radiation generated by the X-ray tube 1104 irradiating X-rays while rotating between the start angle of 0° and end angle of 240°. Note that the half scan is performed in accordance with the following scan condition X1.
Tube voltage: V₁ (kVp)
Beam width: W₁ (mm)
Size of subject body: Body height L₁ (cm), thickness T₁ (cm) of imaging site
Table height: H₁ (cm)

In Embodiment 1, the scan condition X1 includes tube voltage, beam width, subject body size, and table height as scan condition items. Furthermore, the set values for each item are V₁ (kVp) for tube voltage, W₁ (mm) for beam width, L₁ (cm) for body height, T₁ (cm) for thickness of the imaging site, and H₁ (cm) for table height.

After measuring the scattered radiation, the dosimeter 300 is installed in another position. Furthermore, the scattered radiation is re-measured, which is generated by the X-ray tube 1104 irradiating X-rays while rotating between the start angle of 0° and end angle of 240°.

In the same manner, the dosimeter 300 is installed in yet another position, and the scattered radiation, which is generated by the X-ray tube 1104 irradiating X-rays while rotating between the start angle of 0° and end angle of 240°, is repeatedly performed.

Thereby, the scattered radiation is measured, which is generated by the X-ray tube 1104 irradiating X-rays while rotating between the start angle of 0° and end angle of 240°. Furthermore, based on the measurement data obtained from this measurement, a three-dimensional scattered radiation distribution is created that three-dimensionally expresses the distribution of scattered radiation generated by irradiating X-rays while the X-ray tube 1104 rotates between the start angle of 0° and the end angle of 240°.

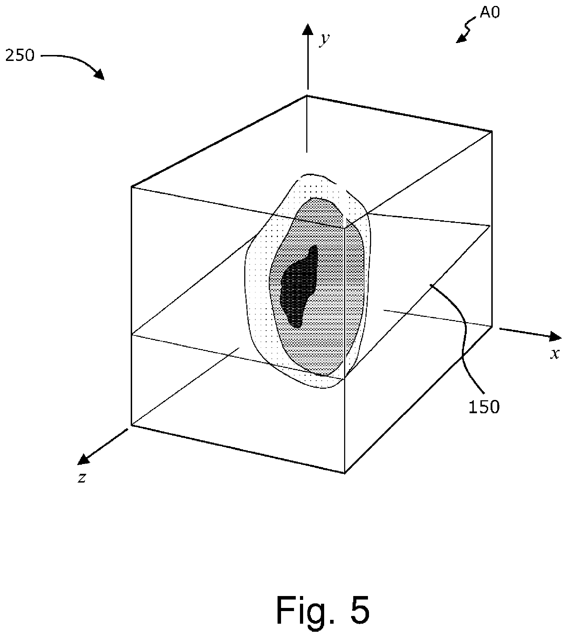

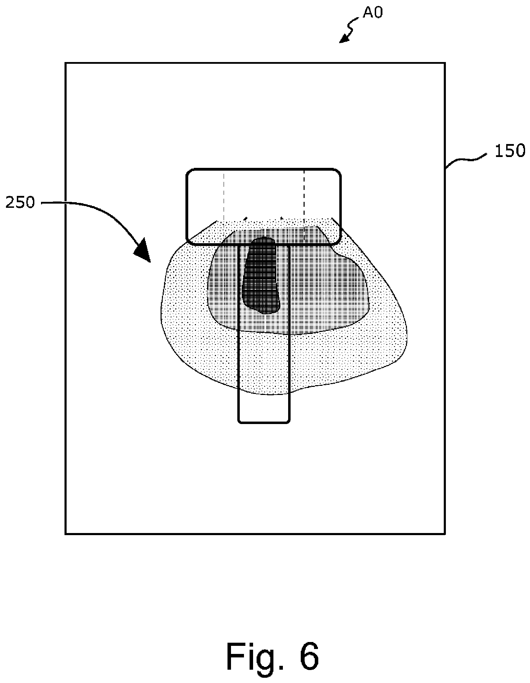

FIG. 5 schematically depicts the three-dimensional scattered radiation distribution A0 when X-rays are irradiated while the X-ray tube 1104 rotates between the start angle of 0° and end angle of 240°. In FIG. 5, the three-dimensional scattered radiation distribution A0 is depicted in the form of a simple cube for convenience of description. The interior of the three-dimensional scattered radiation distribution A0 is indicated by a dot pattern corresponding to the intensity of the scattered radiation. In FIG. 5, the dot pattern is expressed such that regions with higher scattered radiation intensity appear closer to black and regions with lower scattered radiation intensity appear closer to white. FIG. 6 is a diagram schematically depicting a scattered radiation distribution 250 in a cross-section when the three-dimensional scattered radiation distribution A0 depicted in FIG. 5 is cut by a plane 150 perpendicular to the Y-axis. Note that in addition to the scattered radiation distribution 250, the outlines of the gantry and table are also depicted on the plane 150 in order to clarify the positional relationship between the gantry and table and the scattered radiation distribution 250.

In this manner, the three-dimensional scattered radiation distribution A0 when the X-ray tube 1104 irradiates X-rays while the X-ray tube 1104 rotates between the start angle of 0° and end angle of 240° is acquired.

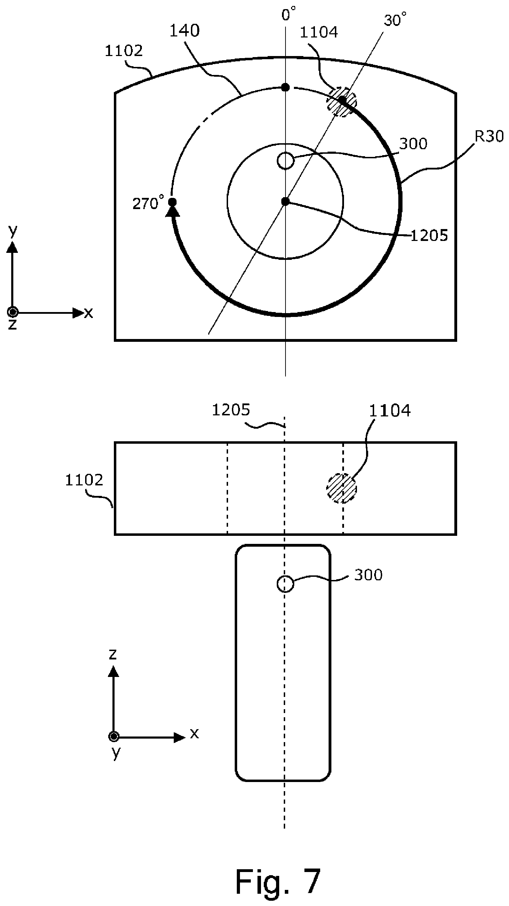

Next, the start angle and end angle of the X-ray tube 1104 are shifted by a prescribed angle α in the clockwise direction, and a half scan for measuring the scattered radiation is performed (see FIG. 7).

FIG. 7 is an explanatory diagram of a half scan when the start angle and end angle of the X-ray tube 1104 are shifted by a prescribed angle α in clockwise direction.

In FIG. 7, the start angle and end angle of the X-ray tube 1104 are shifted by the prescribed angle α in the clockwise direction relative to the start angle and end angle depicted in FIG. 4. In Embodiment 1, the prescribed angle α is α = 30°. Therefore, in FIG. 7, the start angle of the X-ray tube 1104 is 30° and the end angle is 270°. Thus, the X-ray tube 1104 irradiates X-rays while rotating in the angle range R30 between the start angle of 30° and the end angle of 270°.

In FIG. 7, the X-ray tube 1104 begins to irradiate X-rays at the point in time when the X-ray tube 1104 reaches the start angle of 30°. Furthermore, when the X-ray tube 1104 reaches the end angle of 270°, the X-ray tube 1104 finishes irradiating X-rays. Therefore, a half scan is performed by rotating the X-ray tube 1104 through an angle range R30 between the start angle of 30° and the end angle of 270° while irradiating X-rays. The dosimeter 300 measures the scattered radiation generated by the X-ray tube 1104 irradiating X-rays. Note that the half scan of the angle range R30 is performed under the same scan condition X1 as the half scan of the angle range R0 (see FIG. 4).

Furthermore, the dosimeter 300 is installed at another position, and the scattered radiation is again measured, which is generated by the X-ray tube 1104 irradiating X-rays while rotating between the start angle of 30° and end angle of 270°.

In the same manner, the dosimeter 300 is installed in yet another position, and the scattered radiation, which is generated by the X-ray tube 1104 irradiating X-rays while rotating between the start angle of 30° and end angle of 270°, is repeatedly measured.

Thereby, the scattered radiation generated by the X-ray tube 1104 irradiating X-rays while rotating between the start angle of 30° and end angle of 270° is measured. Furthermore, based on the measurement data obtained from this measurement, a three-dimensional scattered radiation distribution is created, where X-rays are irradiated while the X-ray tube 1104 rotates between the start angle of 30° and the end angle of 270°.

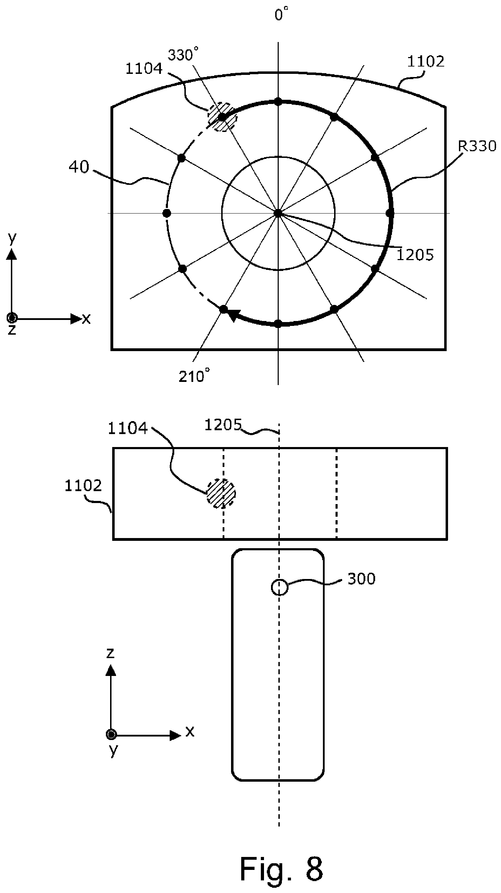

In the same manner, the scattered radiation is measured while shifting the start angle and end angle of the X-ray tube 1104 in the clockwise direction by the prescribed angle α (see FIG. 8).

FIG. 8 is an explanatory diagram of a half scan when the X-ray tube 1104 irradiates X-rays while rotating between a start angle of 330° and an end angle of 210°.

In FIG. 8, the X-ray tube 1104 begins to irradiate X-rays at the point in time when the X-ray tube 1104 reaches the start angle of 330°. Furthermore, when the X-ray tube 1104 reaches the angle of 210°, the X-ray tube 1104 finishes irradiating X-rays. Therefore, a half scan is performed by rotating the X-ray tube 1104 through an angle range R330 between the start angle of 330° and the end angle of 210°. The dosimeter 300 measures the scattered radiation generated by the X-ray tube 1104 irradiating X-rays. The half scan of the angle range R330 is performed under the same scan condition X1 as the half scan of the angle range R0 (see FIG. 4).

Furthermore, the dosimeter 300 is installed at another position, and the scattered radiation is again measured, which is generated by the X-ray tube 1104 irradiating X-rays while rotating between the start angle of 330° and end angle of 210°.

In the same manner, the dosimeter 300 is installed in yet another position, and the scattered radiation, which is generated by the X-ray tube 1104 irradiating X-rays while rotating between the start angle of 330° and end angle of 210°, is repeatedly measured.

Thereby, the scattered radiation generated by the X-ray tube 1104 irradiating X-rays while rotating between the start angle of 330° and end angle of 210° is measured. Furthermore, based on the measurement data obtained from this measurement, a three-dimensional scattered radiation distribution is created that expresses the distribution of scattered radiation generated by irradiating X-rays while the X-ray tube 1104 rotates between the start angle of 330° and the end angle of 210°.

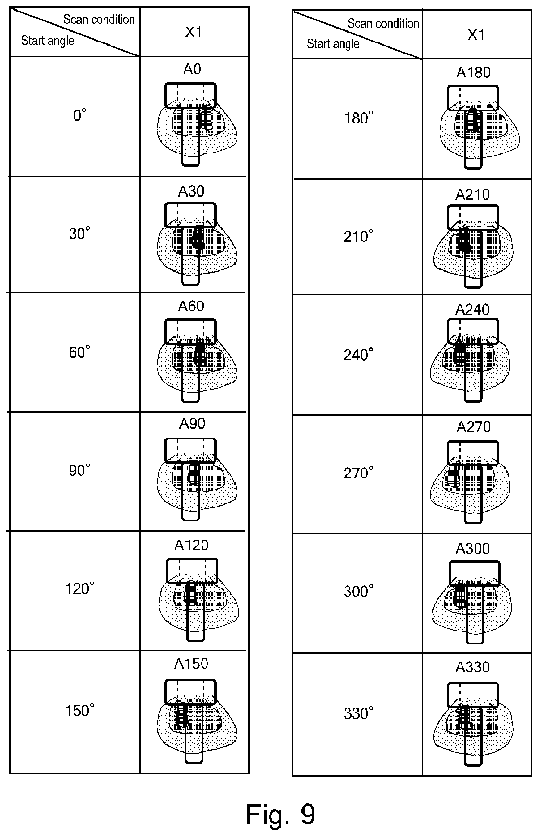

Therefore, a three-dimensional scattered radiation distribution can be obtained when a half scan is performed in accordance with the scan condition X1 while changing the start angle and end angle of the X-ray tube 1104. FIG. 9 is a diagram schematically depicting three-dimensional scattered radiation distributions A0 to A330 when a half scan is performed in accordance with the scan condition X1.

The left half of FIG. 9 depicts schematic views of the three-dimensional scattered radiation distributions A0, A30, A60, A90, A120, and A150 created when the start angle of the X-ray tube 1104 is 0°, 30°, 60°, 90°, 120°, and 150°. The right half of FIG. 9 depicts schematic views of the three-dimensional scattered radiation distributions A180, A210, A240, A270, A300, and A330 created when the start angle of the X-ray tube 1104 is 180°, 210°, 240°, 270°, 300°, and 330°.
Note that in FIG. 9, instead of the three-dimensional scattered radiation distribution, the scattered radiation distribution in the cross-section cut perpendicular to the y-axis of the three-dimensional scattered radiation distribution is depicted due to space limitations. Furthermore, the gantry and table outlines are depicted in the scattered radiation distribution in this cross-section.

In Embodiment 1, the three-dimensional scattered radiation distribution is created by shifting the start angle of the X-ray tube 1104 by 30° in the clockwise direction from 0°. Therefore, the three-dimensional scattered radiation distributions A0 to A330 can be created for 12 start angles of the X-ray tube 104 (0°, 30°, 60°, 90°, 120°, 150°, 180°, 210°, 240°, 270°, 300°, and 330°).

Next, the scan condition X1 is changed and the three-dimensional scattered radiation distribution is obtained for each angle range of the X-ray tube based on another scan condition X2. The scan condition X2 is as follows, for example.
Tube voltage: V₁ (kVp)
Beam width: W₁ (mm)
Size of subject body: Body height Li (cm), thickness T₁ (cm) of imaging site
Table height: H₂ (cm)

The scan condition X2 differs from the scan condition X1 in that the table height is set to H₂, but the other items are the same as in the scan condition X1.

With respect to the scan condition X2, similar to the scan condition X1, a three-dimensional scattered radiation distribution is created for each of the 12 start angles of the X-ray tube 1104.

In the same manner, the three-dimensional scattered radiation distribution is created for each start angle of the X-ray tube 1104 while changing the scan conditions. Therefore, three-dimensional scattered radiation distributions can be obtained for a plurality of scan conditions.

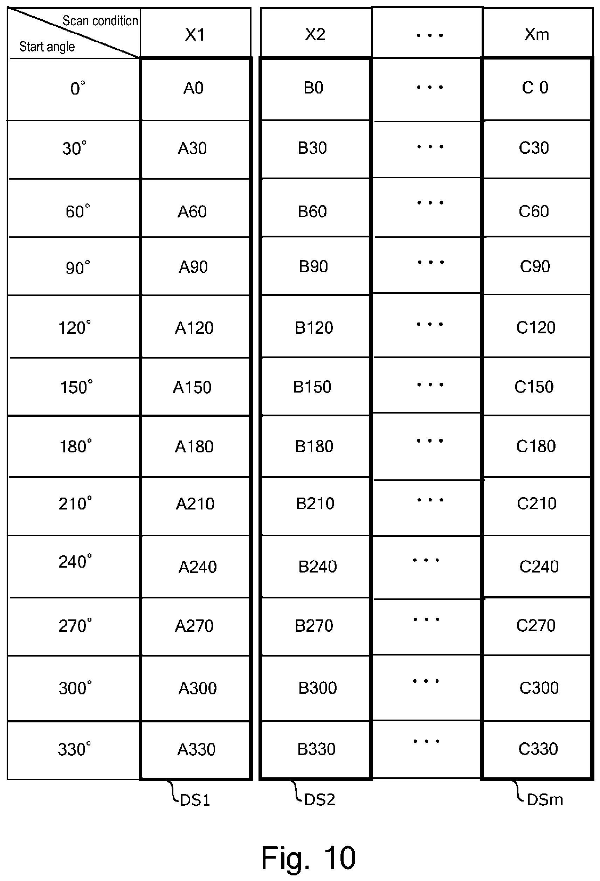

FIG. 10 is a diagram depicting three-dimensional scattered radiation distributions obtained for a plurality of scan conditions X1 to Xm.
FIG. 10 depicts n number of three-dimensional scattered radiation distributions A0 to C330 measured by changing the scan conditions and the start angle of the X-ray tube. The n number of three-dimensional scattered radiation distributions A0 to C330 are divided into scattered radiation distribution data sets DS1 to DSm corresponding to the plurality of scan conditions X1 to Xm. The scattered radiation distribution data sets DS1 to DSm represent scattered radiation distribution data sets obtained by the scan conditions X1 to Xm, respectively. Each scattered radiation distribution data set contains a plurality of three-dimensional scattered radiation distributions corresponding to start angles of 0° to 330°. Note that in FIG. 10, only codes "A0" to "C330" are depicted, while the distribution diagram expressing the three-dimensional scattered radiation distribution is omitted due to space limitations. For example, the scattered radiation distribution data set DS1 corresponding to the scan condition X1 indicates the letters "A0" to "A330" representing the three-dimensional scattered radiation distributions created when the start angle of the X-ray tube 1104 is 0° to 330°. The scattered radiation distribution data set DS2 corresponding to the scan condition X2 indicates the letters "B0" to "B330" representing the three-dimensional scattered radiation distributions created when the start angle of the X-ray tube 1104 is 0° to 330°. Furthermore, the scattered radiation distribution data set DSm corresponding to the scan condition Xm indicates the letters "C0" to "C330" representing the three-dimensional scattered radiation distributions created when the start angle of the X-ray tube 1104 is 0° to 330°.

These three-dimensional scattered radiation distributions A0 to C330 are stored in the storage device 218 or in an external storage device accessible by the CT system 100 (see FIG. 1 or FIG. 2) as three-dimensional scattered radiation distributions when a half scan is performed by the CT system 100. Each three-dimensional scattered radiation distribution is stored in association with the scan condition and the start angle of the X-ray tube. For example, the three-dimensional scattered radiation distribution A180 is stored in association with the scan condition X1 and a start angle of 180° of the X-ray tube.

The CT system 100 uses the three-dimensional scattered radiation distributions A0 to C330 prepared as described above to allow the surgeon 101 during CT-guided puncture to be aware of the level of the scattered radiation intensity at the standing position of the surgeon 101. Hereinafter, a description is given of the flow for allowing the surgeon 101 to be aware of the scattered radiation intensity during the CT-guided puncture.

FIG. 11 is a flowchart of the CT-guided puncture.
In step ST1, a diagnostic scan is performed for acquiring a CT image of an imaging site of the subject body 112. A processor in the computer 216 or image reconstructor 230 acquires a CT image of the imaging site based on the data acquired by the diagnostic scan. After acquiring the CT image, the flow proceeds to step ST2.

In step ST2, the surgeon performing the CT-guided puncture confirms the position of the target (e.g., lesion) based on the CT image and determines the puncture position, the path of needle puncture, and the like. After determining the puncture path and the like, the flow proceeds to step ST3.

In step ST3, the CT-guided puncture is initiated.

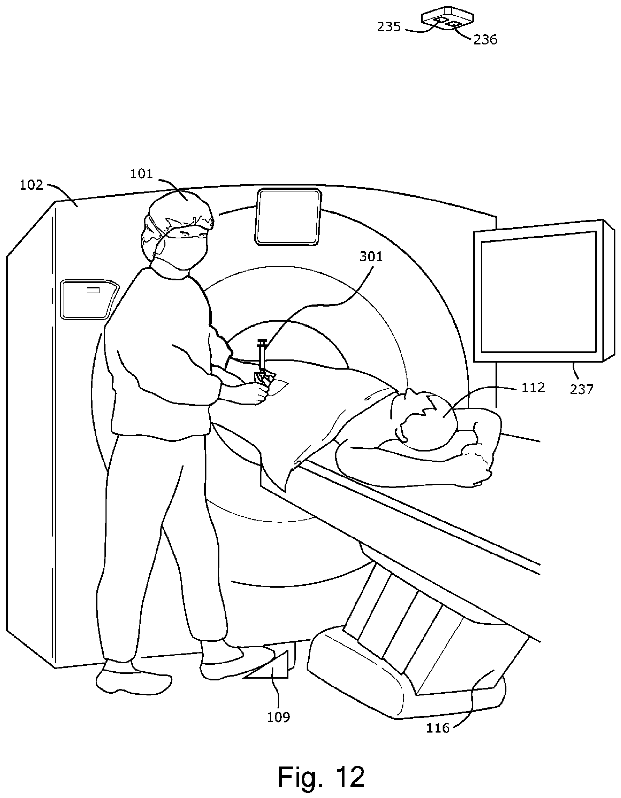

FIG. 12 is a diagram depicting the surgeon 101 performing the CT-guided puncture using the CT system 100.
While performing the puncture, the surgeon 101 must confirm the positional relationship between a tip end of a needle 301 and the target (e.g., lesion) and the orientation of the tip end of the needle 301. The surgeon 101 acquires a CT image of the puncture site during the puncture to perform the confirmation work. The surgeon 101 prepares a scan for acquiring a CT image and confirms that the scan may be performed. When the scan is ready, the surgeon 101 operates a hand controller (not depicted) and presses a confirm button on the hand controller in step ST4 to make the CT system 100 recognize that the scan is ready to be performed.

When the confirm button is pressed, the hand controller outputs a confirmation signal to the CT system 100 indicating that it is confirmed that the puncture site may be scanned.

When the CT system 100 receives the confirmation signal, the gantry motor controller 212 rotates the X-ray tube 104 and detector 108 in accordance with the instruction from the processor in the computer 216.

In step ST5, the surgeon 101 steps on the foot pedal 109 to start the first half scan. When the foot pedal 109 is depressed, the flow proceeds to step ST6.

In step ST6, the first half scan is performed.

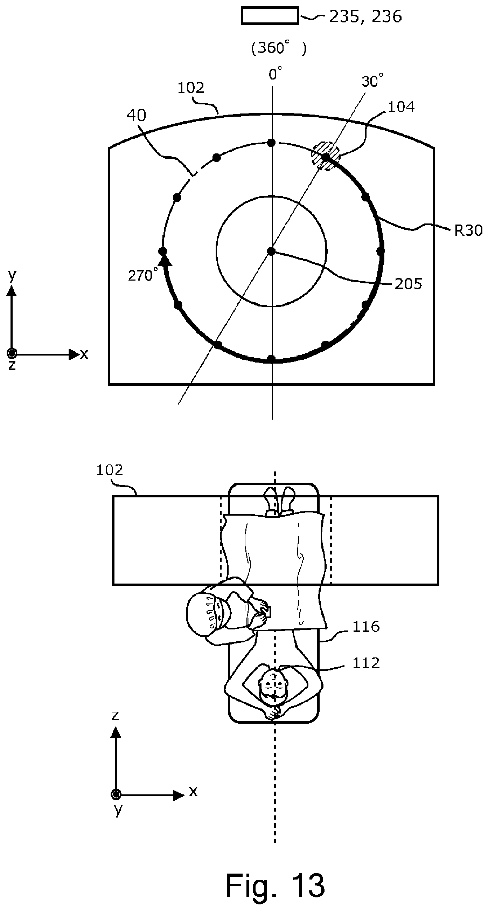

FIG. 13 is an explanatory diagram of the first half scan.
A front surface view of the gantry 102 of the CT system 100 is depicted on the upper side of FIG. 13, and an upper surface view of the gantry 102 and the table 116 is depicted on the lower side of FIG. 13.

When the foot pedal is depressed, the computer 216 outputs a signal to the X-ray controller 210 (see FIG. 2) indicating that the foot pedal has been depressed. When the X-ray controller 210 receives the signal indicating that the foot pedal is depressed, it determines that a trigger for starting a half scan of the subject body 112 has occurred and controls the X-ray tube 104 such that the half scan is started.

FIG. 13 depicts a position of the X-ray tube 104 at a point in time when X-ray irradiation begins from the X-ray tube 104 in the first half scan. Herein, it is assumed that X-ray irradiation from the X-ray tube 104 starts when the tube 104 reaches an angle of 30°.
Therefore, the processor of the computer 216 determines that an angle of 30° is the start angle, which represents the angle of the X-ray tube when starting the half scan.

Once X-ray irradiation begins at the start angle of 30°, the half scan is performed from the start angle of 30° to a rotation of 180° + fan angle. In Embodiment 1, the fan angle is 60°, and therefore, a half scan is performed until the X-ray tube 104 rotates 180° + 60° = 240° from the start angle of 30°. Therefore, the processor of the computer 216 determines that an angle of 270° is the end angle, which represents the angle of the X-ray tube at the end of the half scan.

The X-ray tube 104 terminates X-ray irradiation at a point in time when the end angle of 270° is reached. In this manner, the first half scan is completed.

On the other hand, if the confirm button is pressed in step ST4, the processor of the computer 216 identifies, in step ST7, the position and shape of the surgeon 101 in the scan room based on a camera image (optical image) obtained by the camera 235 at the point in time when the confirm button is pressed. After identifying the position and shape of the surgeon 101, the flow proceeds to step ST8.

In step ST8, the processor of the computer 216 determines the three-dimensional scattered radiation distribution corresponding to the half scan performed in step ST6 based on the three-dimensional scattered radiation distributions A0 to C330 (see FIG. 10) stored in the storage device. A method for determining the three-dimensional scattered radiation distribution in step ST8 will be described below.

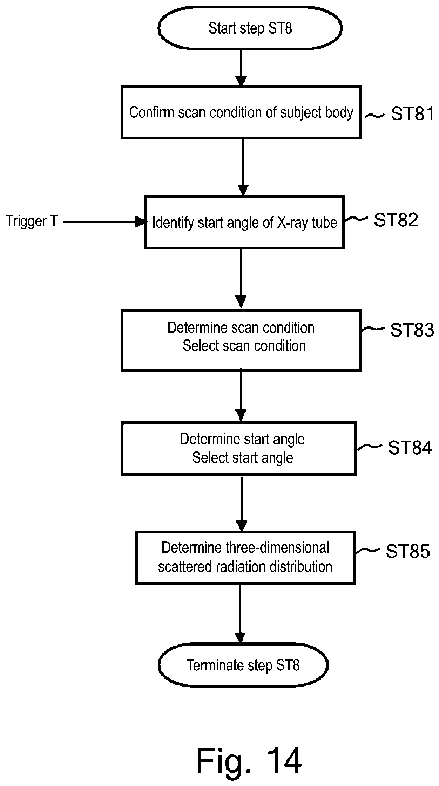

FIG. 14 is an explanatory diagram of step ST8.
In step ST81, the processor of the computer 216 confirms a scan condition B of the subject body 112. In Embodiment 1, the scan condition B for the subject body 112 is, for example, as follows.
Tube voltage: V₁ (kVp)
Beam width: W₁ (mm)
Size of subject body: Body height L₁ (cm), thickness T₁ (cm) of imaging site
Table height: H₁ (cm)

The tube voltage is a value determined by the CT system used to capture an image of the subject body 112. The beam width and table height are values determined at the point in time when the subject body 112 is ready for puncture. The size of the subject body 112 is a value obtained by measuring the physique before the diagnostic scan, by obtaining patient information from a hospital information system (HIS), or the like. Therefore, before the half scan of the subject body 112, the set values for each item of the scan condition B for the subject body 112 have been determined, and thus the processor can confirm the scan condition B for the subject body 112. After confirming the scan condition B, the flow proceeds to step ST82.

In step ST82, the processor of the computer 216 identifies the start angle of the X-ray tube 104 when performing the half scan in step ST6. In Embodiment 1, the processor identifies the angle of the X-ray tube 104 as the start angle of the X-ray tube 104 at the point in time when the trigger T for starting the half scan is generated by the foot pedal being depressed in step ST5. Herein, as depicted in FIG. 13, the angle of the X-ray tube 104 at the point in time when the trigger T occurs is 30°. Therefore, the processor identifies the start angle of the X-ray tube 104 as 30°. Note that the start angle of the X-ray tube may be set to the point in time when the trigger is generated by an action other than depressing the foot pedal. After identifying the start angle of the X-ray tube 104, the flow proceeds to step ST83.

In step ST83, the processor of the computer 216 determines whether the scan conditions X1 to Xm (see FIG. 10) include a scan condition that matches the scan condition B of the subject body. Herein, it is assumed that the scan condition B of the subject body matches the scan condition X1. Therefore, the processor determines that scan condition X1 to Xm include the scan condition X1 that matches the scan condition B of the subject body. In this case, the processor selects the scan condition X1 that matches the scan condition B of the subject body from the scan conditions X1 to Xm.

In step ST84, the processor of the computer 216 determines whether the start angles 0° to 330° includes a start angle that matches the start angle identified in step ST82. In step ST82, the start angle is identified as 30°. Therefore, it is determined that the start angle of 30° identified in step ST82 is included in the start angle of 0° to 330°. In this case, the processor selects the start angle of 30° from the plurality of start angles of 0° to 330°.

In step ST85, the processor of the computer 216 determines the three-dimensional scattered radiation distribution corresponding to the half scan based on the results of the determinations and selections in steps ST83 and ST84. This determination is performed as follows.
The processor selects the three-dimensional scattered radiation distribution corresponding to the scan condition X1 selected in step ST83 and the start angle of 30° selected in step ST84 from the n number of three-dimensional scattered radiation distributions A0 to C330 (see FIG. 10). Referring to FIG. 10, the three-dimensional scattered radiation distribution A30 is associated with the scan condition X1 and start angle of 30°. Therefore, the processor of the computer 216 selects the three-dimensional scattered radiation distribution A30 as the three-dimensional scattered radiation distribution corresponding to the scan condition X1 and start angle of 30° from the three-dimensional scattered radiation distributions A0 to C330.
The three-dimensional scattered radiation distribution A30 selected in this manner is determined as the three-dimensional scattered radiation distribution corresponding to the half scan performed in step ST6, and thus the flow of step ST8 is terminated.

Returning to FIG. 11, the description is continued.
After selecting the three-dimensional scattered radiation distribution A30 in step ST8, the flow proceeds to step ST9.

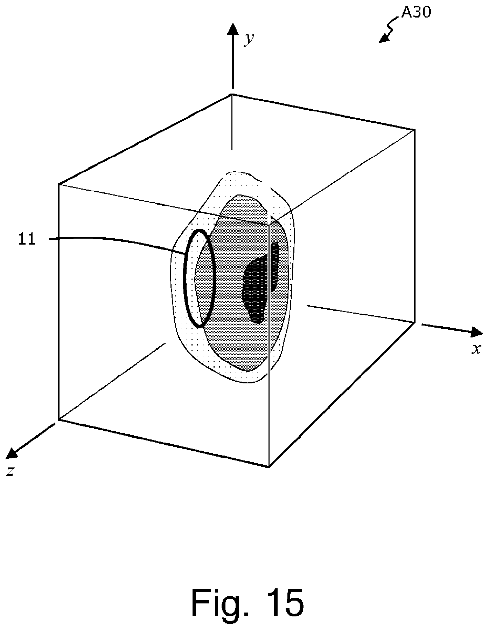

FIG. 15 is an explanatory diagram of step ST9.
In step ST9, the processor of the computer 216 identifies the scattered radiation distribution 11 in the region where the surgeon 101 is present from the three-dimensional scattered radiation distribution A30 selected in step ST8, based on the position and shape of the surgeon 101. In FIG. 15, the scattered radiation distribution 11 in the region where the surgeon 101 is present is depicted by the area enclosed by the oval. After identifying the scattered radiation distribution 11 from the three-dimensional scattered radiation distribution A30, the flow proceeds to step ST10.

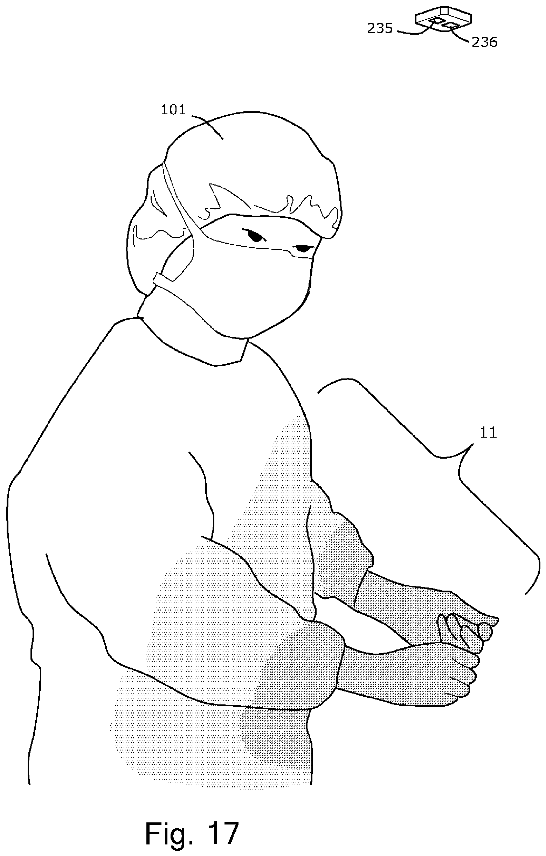

In step ST10, the scattered radiation distribution 11 identified from the three-dimensional scattered radiation distribution A30 is displayed.

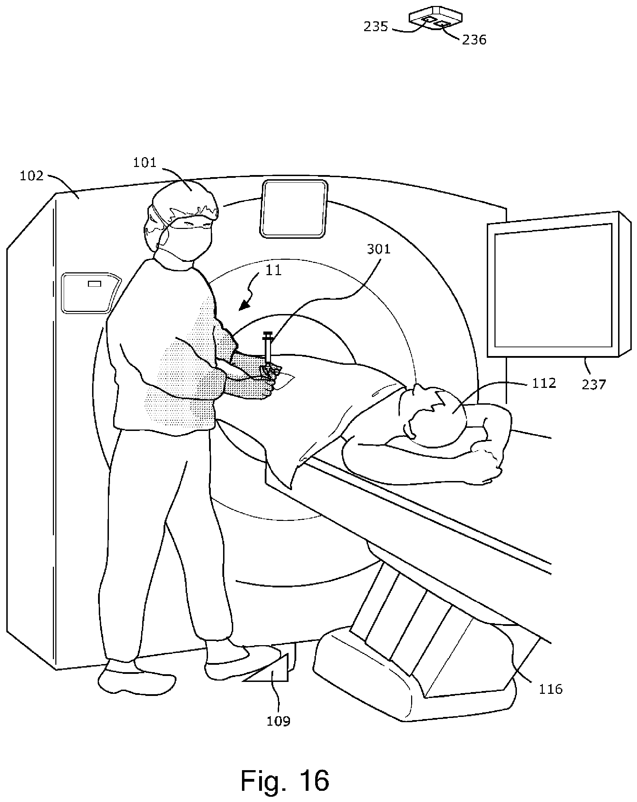

FIG. 16 is an explanatory diagram of step ST10.
The processor of the computer 216 projects the identified scattered radiation distribution 11 from the three-dimensional scattered radiation distribution A30 onto the surgeon 101 using the projection device 236. Therefore, the scattered radiation distribution 11 can be displayed on the body surface of the surgeon 101. FIG. 17 is an enlarged view of the surgeon 101 on which a scattered radiation distribution 11 is projected. In Embodiment 1, the scattered radiation distribution 11 is indicated by a dot pattern corresponding to the scattered radiation intensity. In FIG. 17, the dot pattern is set such that regions with higher scattered radiation intensity appear closer to black and regions with lower scattered radiation intensity appear closer to white.

The scattered radiation distribution 11 is projected on the surgeon's own body surface, and therefore, the surgeon 101 can visually recognize, during the puncture, the level of exposure to the scattered radiation generated by the first half scan. Note that in FIG. 16 and FIG. 17, the scattered radiation distribution 11 is displayed as a dot pattern corresponding to the scattered radiation intensity, but arbitrary means can be used so long as differences in the intensity of the scattered radiation can be displayed. For example, the scattered radiation distribution may be displayed with different color hue, saturation, and/or brightness depending on the intensity of the scattered radiation.

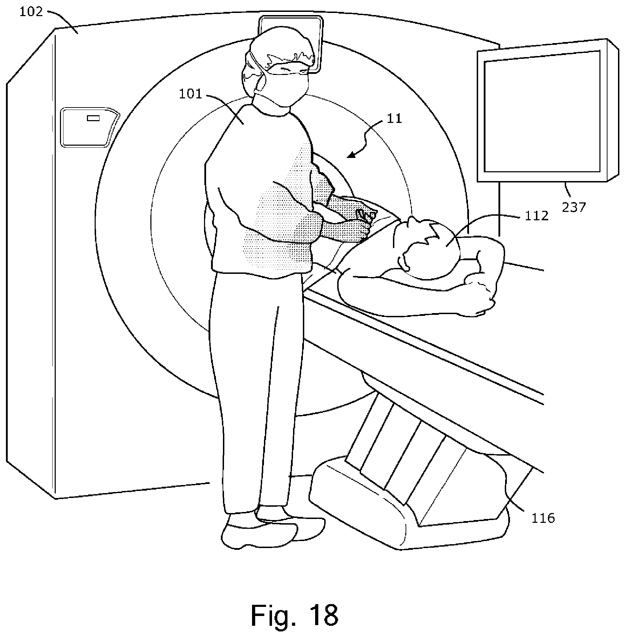

Furthermore, the surgeon 101 may change the standing position, if necessary, after the half scan is completed. If the surgeon 101 changes the standing position, the processor adjusts the projected position of the scattered radiation distribution 11 according to the standing position of the surgeon 101 such that there is no shift in the projection position of the scattered radiation distribution 11 (see FIG. 18).

FIG. 18 is a diagram depicting the projection position of the scattered radiation distribution 11 when the surgeon 101 changes the standing position.
The processor projects the scattered radiation distribution 11 to the surgeon 101 who has changed the standing position. Therefore, even if the surgeon 101 changes the standing position after the half scan is completed, the scattered radiation distribution 11 can be projected in the correct position with respect to the surgeon 101. Thus, the surgeon 101 can correctly recognize the exposure dose to each part of the surgeon 101 from the half scan, even if the standing position is changed during the puncture.
After the half scan is performed in step ST6, the flow proceeds to step ST11.

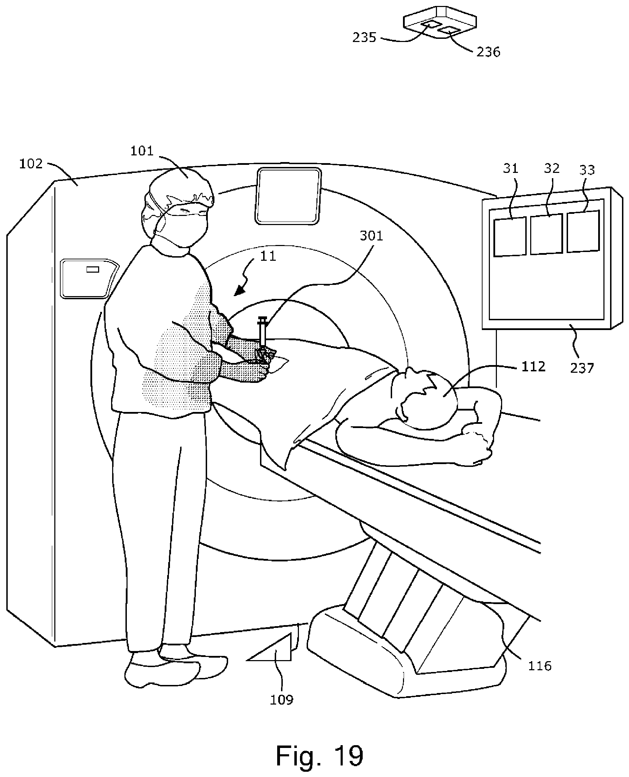

In step ST11, the processor of the image reconstructor 230 reconstructs a CT image of a puncture portion based on data obtained by the half scan. Furthermore, the processor displays the reconstructed CT image on an interventional monitor 237. FIG. 19 is a diagram schematically depicting CT images 31, 32, and 33 displayed on the intervention monitor 237. After displaying the CT images 31, 32, and 33, the flow proceeds to step ST12.

In step ST12, the surgeon 101 confirms the position of the needle 301 while viewing the CT images 31, 32, and 33 to determine if the tip end of the needle 301 has reached a desired position. If the needle 301 has reached the desired position, the flow proceeds to step ST13, tissue is collected, and then the flow ends. On the other hand, if the tip end of the needle 301 has not reached the desired position, the flow returns to step ST4. Herein, it is determined that the tip end of the needle 301 has not yet reached the desired position. Therefore, the flow returns to step ST4.

In step ST4, the surgeon 101 acquires a CT image of the puncture site during the puncture to confirm the positional relationship between the tip end of the advanced needle 301 and the target as well as the orientation of the tip end of the needle 301. The surgeon 101 prepares a scan for acquiring a CT image and confirms that the scan may be performed. When the scan is ready, the surgeon 101 operates a hand controller (not depicted) and presses a confirm button on the hand controller to make the CT system 100 recognize that the scan is ready to be performed.

When the confirm button is pressed, the hand controller outputs a confirmation signal to the CT system 100 indicating that it is confirmed that the puncture site may be scanned. After the confirm button is pressed, the flow proceeds to step ST5.

In step ST5, the surgeon 101 steps on the foot pedal 109 to start the second half scan. When the foot pedal 109 is depressed, the flow proceeds to step ST6.

In step ST6, the second half scan is performed.

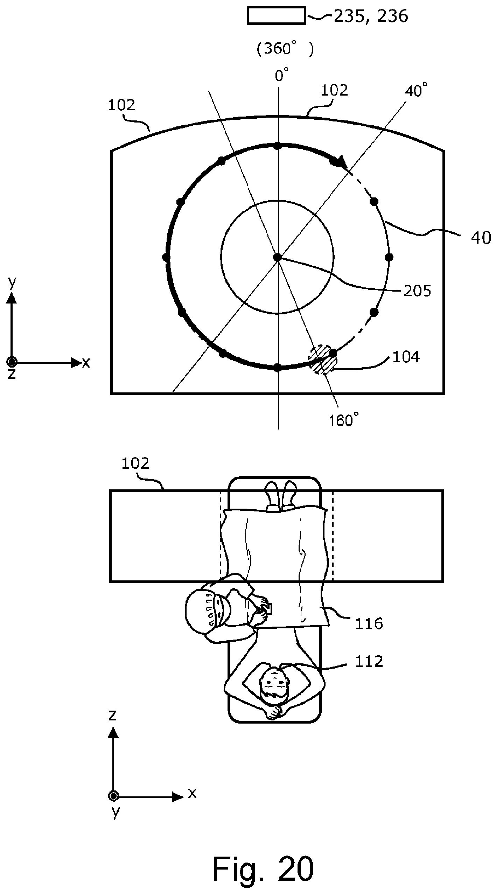

FIG. 20 is an explanatory diagram of the second half scan.
When the foot pedal is depressed, the X-ray controller determines that a trigger T has occurred for starting a half scan and controls the X-ray tube 104 such that the half scan is started.

FIG. 20 depicts the angle of the X-ray tube 104 at a point in time when the trigger T for starting the second half scan occurs. Herein, the X-ray tube 104 has reached an angle of 160° at the point in time when the trigger T occurs. Therefore, X-ray irradiation from the X-ray tube 104 begins when the X-ray tube 104 reaches the angle of 160°.

Once the X-ray tube 104 starts X-ray irradiation at the start angle of 160°, the half scan is performed from the start angle of 160° to a rotation of 180° + fan angle. In Embodiment 1, the fan angle is 60°, and therefore, the half scan is performed until the X-ray tube 104 rotates 180° + 60° = 240° from the start angle of 160°. The X-ray tube 104 terminates X-ray irradiation at a point in time when the end angle of 40° is reached. In this manner, the second half scan is completed.

On the other hand, if the confirm button is pressed in step ST4, the processor of the computer 216 identifies, in step ST7, the position and shape of the surgeon 101 in the scan room based on a camera image acquired by the camera 235 at the point in time when the confirm button is pressed. After identifying the position and shape of the surgeon 101, the flow proceeds to step ST8.

In step ST8, the processor of the computer 216 determines the three-dimensional scattered radiation distribution corresponding to the second half scan performed in step ST6 based on the three-dimensional scattered radiation distributions A0 to C330 (see FIG. 10) stored in the storage device. A method for determining the three-dimensional scattered radiation distribution in step ST8 will be described below while referring to the flow of FIG. 14.

In step ST81, the processor of the computer 216 confirms a scan condition B of the subject body 112. Furthermore, in step ST82, the processor of the computer 216 identifies the start angle of the X-ray tube 104 when performing the second half scan. The processor identifies the angle of 160° (see FIG. 20) of the X-ray tube 104 at the point in time when the trigger T for starting the second half scan occurs as the start angle of the X-ray tube 104. After identifying the start angle of the X-ray tube 104, the flow proceeds to step ST83.

In step ST83, the processor of the computer 216 determines whether the scan conditions X1 to Xm include a scan condition that matches the scan condition B of the subject body. As described above, the scan condition B of the subject body 112 matches the scan condition X1. Therefore, the processor selects the scan condition X1 that matches the scan condition B of the subject body from the scan conditions X1 to Xm.

In step ST84, the processor of the computer 216 determines whether the start angles 0° to 330° includes a start angle that matches the start angle identified in step ST82. In step ST82, the start angle is identified as 160°. Therefore, it is determined that the start angle of 160° identified in step ST82 is not included in the start angle of 0° to 330°. In this case, the processor selects a start angle of 150° that is closest to the starting angle of 160° from the plurality of start angles 0° to 330°.

In step ST85, the processor of the computer 216 determines the three-dimensional scattered radiation distribution corresponding to the second half scan based on the results of the determinations and selections in steps ST83 and ST84. This determination is performed as follows.

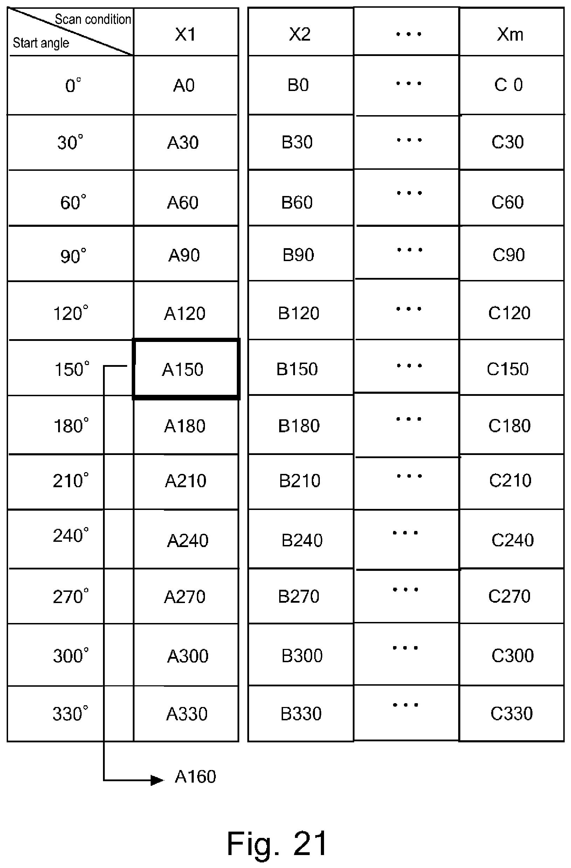

FIG. 21 is an explanatory diagram of step ST85.
The processor selects the three-dimensional scattered radiation distribution A150 corresponding to the scan condition X1 selected in step ST83 and the start angle of 150° selected in step ST84 from the n number of three-dimensional scattered radiation distributions A0 to C330. However, the start angle of 150° corresponding to the three-dimensional scattered radiation distribution A150 is different from the start angle of 160° identified in step ST82. Therefore, the processor corrects the three-dimensional scattered radiation distribution A150 based on the angular difference between the start angle of 150° and the start angle of 160°. In FIG. 21, the three-dimensional scattered radiation distribution obtained by correcting the three-dimensional scattered radiation distribution A150 is indicated by the code "A160".

Note that in FIG. 21, the three-dimensional scattered radiation distribution A160 is obtained by correcting the three-dimensional scattered radiation distribution A150. However, the three-dimensional scattered radiation distribution A160 may be obtained by correcting a different three-dimensional scattered radiation distribution (e.g., three-dimensional scattered radiation distribution A180) than the three-dimensional scattered radiation distribution A150.

The three-dimensional scattered radiation distribution A160 obtained in this manner is determined as the three-dimensional scattered radiation distribution corresponding to the second half scan performed in step ST6. Once the three-dimensional scattered radiation distribution A160 is obtained, the flow of step ST8 is terminated.

Returning to FIG. 11, the description is continued.
After obtaining the three-dimensional scattered radiation distribution A160, the flow proceeds to step ST9.

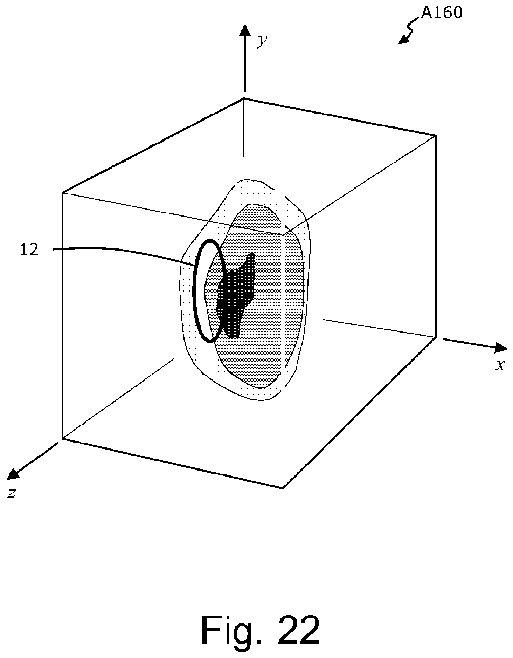

FIG. 22 is an explanatory diagram of step ST9.
In step ST9, the processor of the computer 216 identifies the scattered radiation distribution 12 in the region where the surgeon 101 is present from the three-dimensional scattered radiation distribution A160 obtained in step ST8, based on the position and shape of the surgeon 101. In FIG. 22, the scattered radiation distribution 12 in the region where the surgeon 101 is present is depicted by the area enclosed by the oval. After identifying the scattered radiation distribution 12, the flow proceeds to step ST10.

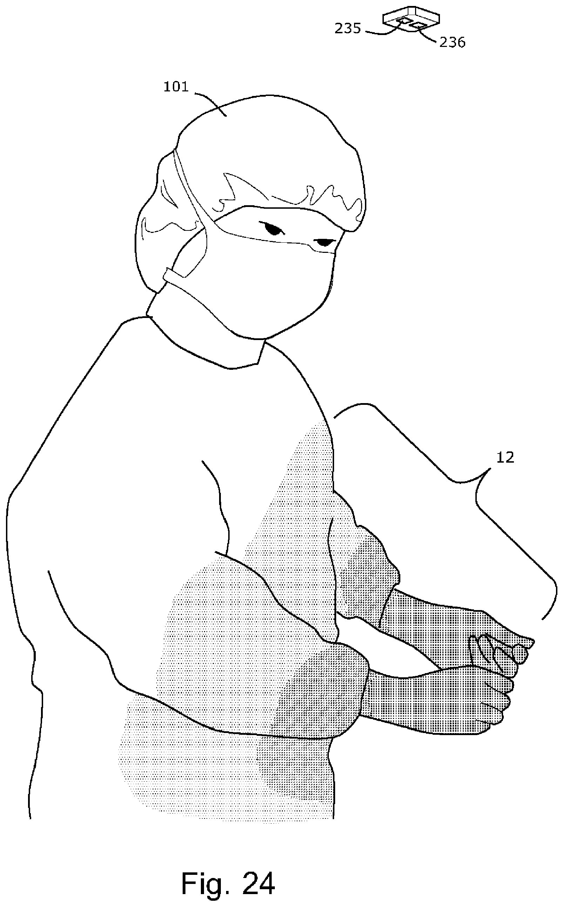

In step ST10, the scattered radiation distribution 12 identified from the three-dimensional scattered radiation distribution A160 is displayed.

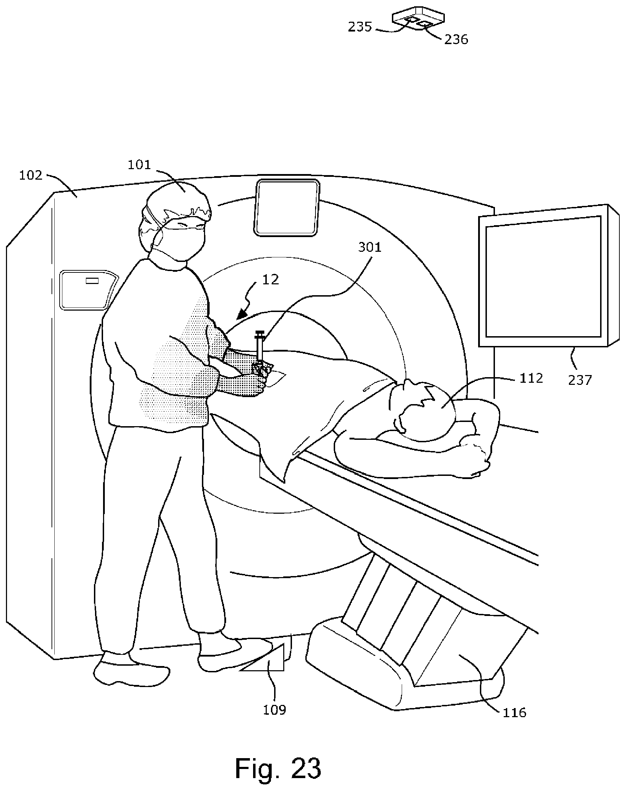

FIG. 23 is an explanatory diagram of step ST10.
The processor of the computer 216 projects the identified scattered radiation distribution 12 from the three-dimensional scattered radiation distribution A160 onto the surgeon 101 using the projection device 236. Therefore, the scattered radiation distribution 12 can be displayed on the body surface of the surgeon 101. FIG. 24 is an enlarged view of the surgeon 101 on which a scattered radiation distribution 12 is projected. The scattered radiation distribution 12 is projected on the surgeon's own body surface, and therefore, the surgeon 101 can visually recognize, during the puncture, the level of exposure to the scattered radiation generated by the second half scan.

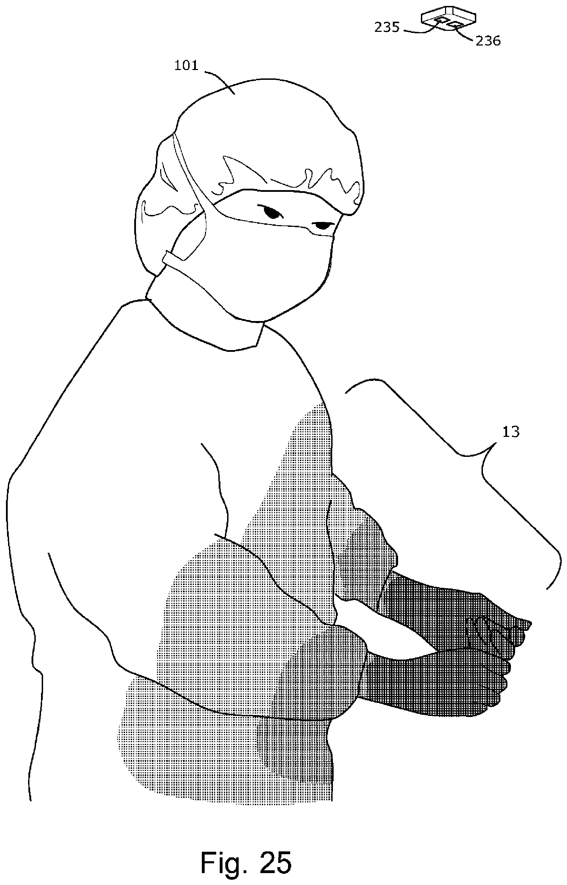

Note that in FIG. 23 and FIG. 24, the scattered radiation distribution 12 from the second half scan is depicted. However, in Embodiment 1, the surgeon 101 is exposed to exposure expressed by the scattered radiation distribution 11 depicted in FIG. 17 when the first half scan is performed. Therefore, the cumulative scattered radiation distribution may be projected onto the surgeon 101 to allow the surgeon 101 to be visually aware of the cumulative exposure to the surgeon 101 from performing the first and second half scans (see FIG. 25).

FIG. 25 is an explanatory diagram of the cumulative scattered radiation distribution.
A cumulative scattered radiation distribution 13 represents the scattered radiation distribution 11 from the first half scan (see FIG. 16 and FIG. 17) superimposed on the scattered radiation distribution 12 from the second half scan (see FIG. 23 and FIG. 24). By projecting the cumulative scattered radiation distribution 13 onto the surgeon themselves, the surgeon 101 can visually recognize how much exposure has accumulated due to scattered radiation at this point in time.

Furthermore, after the second half scan is terminated, if the surgeon 101 changes the standing position, the processor adjusts the projected position of the cumulative scattered radiation distribution 13 according to the standing position of the surgeon 101 such that there is no shift in the projection position of the cumulative scattered radiation distribution 13. By adjusting the projection position of the cumulative scattered radiation distribution 13 according to the standing position of the surgeon 101, the surgeon 101 can correctly recognize the cumulative exposure dose at each site of the surgeon 101, even if the standing position is changed during the puncture.
After the half scan is performed in step ST6, the flow proceeds to step ST11.

In step ST11, the processor of the image reconstructor 230 reconstructs a CT image of a puncture portion based on data obtained by the half scan. Furthermore, the processor displays the reconstructed CT image on an interventional monitor 237.

In step ST12, the surgeon 101 confirms the position of the needle 301 while viewing a CT image displayed on the intervention monitor 237 to determine if the tip end of the needle 301 has reached a desired position. If the needle 301 has reached the desired position, the flow proceeds to step ST13, tissue is collected, and then the flow ends. On the other hand, if the tip end of the needle 301 has not reached the desired position, the flow returns to step ST4. Herein, it is determined that the tip end of the needle 301 has not yet reached the desired position. Therefore, the flow returns to step ST4.

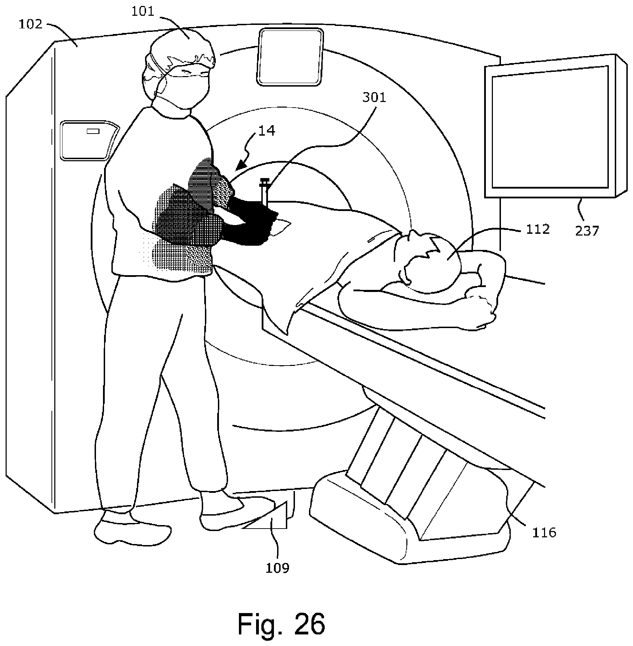

In the same manner, steps ST4 to ST12 are repeated until the tip end of the needle 301 is determined to be at the desired position in step ST12. Therefore, each time a half scan is performed, the scattered radiation distribution (see FIG. 17 and FIG. 24) or cumulative scattered radiation distribution (see FIG. 25) is projected onto the surgeon 101 in step ST10, such that the surgeon 101 can visually recognize the level of exposure to scattered radiation. FIG. 26 is a diagram schematically depicting a cumulative scattered radiation distribution 14 expressing cumulative exposure of the scattered radiation on the surgeon 101 between the start and end of the CT-guided puncture. By projecting the cumulative scattered radiation distribution 14 onto the surgeon 101, the surgeon 101 can visually recognize the level of scattered radiation accumulated from performing a single CT-guided puncture. Note that the exposure dose received by each site of the surgeon 101 during the single CT-guided puncture may be calculated, and the calculated value of the exposure dose for each site of the surgeon may be displayed on the display device.

In step ST12, if the tip end of the needle 301 is determined to be in the desired position, tissue is collected in step ST13 and the flow ends.

In Embodiment 1, steps ST4 to ST12 are repeated until the tip end of the needle 301 is determined to be at the desired position in step ST12. Furthermore, each time a half scan is performed, the three-dimensional scattered radiation distribution corresponding to the half scan performed is determined in step ST8. After the three-dimensional scattered radiation distribution is determined in step ST8, the scattered radiation distribution in the region where the surgeon is present is identified from the determined three-dimensional scattered radiation distribution in step ST9. Furthermore, in step ST10, the identified scattered radiation distribution is displayed. Therefore, it is possible to allow the surgeon 101 to be visually aware of the level of exposure of the surgeon 101 to scattered radiation.

Furthermore, in Embodiment 1, each time a half scan is performed, a cumulative scattered radiation distribution, which represents how much exposure has accumulated on the surgeon 101, can also be projected onto the surgeon 101. Therefore, the surgeon 101 can also visually recognize how much exposure was accumulated from the single CT-guided puncture performed this time.

Note that in Embodiment 1, when determining the three-dimensional scattered radiation distribution corresponding to the half scan in step ST8, the three-dimensional scattered radiation distribution is determined in accordance with the flow depicted in FIG. 14. Furthermore, Embodiment 1 describes a case in which it is determined that the scan conditions X1 to Xm include a scan condition that matches the scan condition B of the subject body 112 in step ST83 of the flow depicted in FIG. 14. However, depending on the set value of the scan condition B for the subject body 112, the scan conditions X1 to Xm may not include a scan condition that matches the scan condition B for the subject body 112. Therefore, the flow in FIG. 14 is described below, in which the scan conditions X1 to Xm do not include a scan condition that matches the scan condition B for the subject body 112.

In step ST81, the processor of the computer 216 confirms the scan condition B of the subject body 112. Herein, the scan condition B for the subject body 112 is as follows.
Tube voltage: V₁ (kVp)
Beam width: W₁ (mm)
Size of subject body: Body height Li (cm), thickness T₁ (cm) of imaging site
Table height: H₃ (cm)

Furthermore, in step ST82, the processor of the computer 216 identifies the start angle of the X-ray tube 104 when performing the half scan. Herein, it is assumed that the start angle of the X-ray tube is "30°". After identifying the start angle of the X-ray tube 104, the flow proceeds to step ST83.

In step ST83, the processor of the computer 216 determines whether the scan conditions X1 to Xm include a scan condition that matches the scan condition B of the subject body. Herein, as described above, it is assumed that the scan conditions X1 to Xm do not include the scan condition X1 that matches the scan condition B of the subject body. In this case, the processor selects one scan condition from the scan conditions X1 to Xm. The processor selects, for example, the scan condition X1.

In step ST84, the processor of the computer 216 determines whether the start angles 0° to 330° includes a start angle that matches the start angle identified in step ST82. In step ST82, the start angle is identified as 30°. Therefore, it is determined that the start angle of 30° identified in step ST82 is included in the start angle of 0° to 330°. In this case, the processor selects the start angle of 30° from the plurality of start angles of 0° to 330°.

In step ST85, the processor of the computer 216 determines the three-dimensional scattered radiation distribution corresponding to the second half scan based on the results of the determinations and selections in steps ST83 and ST84. This determination is performed as follows.

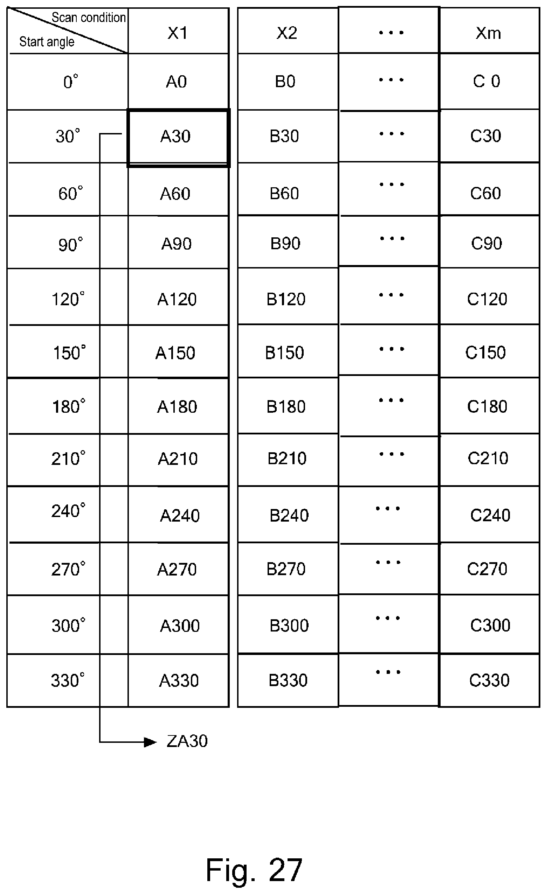

FIG. 27 is an explanatory diagram of step ST85.
The processor selects the three-dimensional scattered radiation distribution A30 corresponding to the scan condition X1 selected in step ST83 and the start angle of 30° selected in step ST84 from the n number of three-dimensional scattered radiation distributions A0 to C330. However, the scan condition X1 corresponding to the three-dimensional scattered radiation distribution A30 is different from the scan condition B of the subject body. Therefore, the processor compares the scan condition X1 with the scan condition B of the subject body and calculates the difference between the set values of each item in these two scan conditions. The scan condition X1 and scan condition B for the subject body 112 are as follows.

### (1) Scan condition X1

Tube voltage: V₁ (kVp)
Beam width: W₁ (mm)
Size of subject body: Body height Li (cm), thickness T₁ (cm) of imaging site
Table height: H₁ (cm)

### (2) Scan condition B of subject body 112

Tube voltage: V₁ (kVp)
Beam width: W₁ (mm)
Size of subject body: Body height Li (cm), thickness T₁ (cm) of imaging site
Table height: H₃ (cm)

When the scan condition X1 is compared with the scan condition B for the subject body, the tube voltage, beam width, and subject body size are the same. However, the table heights are different. Therefore, the processor calculates the difference ΔH (=H₃-H₁) in table heights. Furthermore, the processor corrects the three-dimensional scattered radiation distribution A30 based on the difference ΔH. In FIG. 27, the three-dimensional scattered radiation distribution obtained by correcting the three-dimensional scattered radiation distribution A30 is indicated by the code "ZA30".

The three-dimensional scattered radiation distribution ZA30 obtained in this manner is determined as the three-dimensional scattered radiation distribution corresponding to the half scan, and the flow of step ST8 is terminated. Therefore, even if the scan conditions X1 to Xm do not include a scan condition that matches the scan condition B of the subject body, the three-dimensional scattered radiation distribution ZA30 corresponding to the scan condition B of the subject body can be obtained by correcting the three-dimensional scattered radiation distribution. Note that in the aforementioned description, only the heights of the table are different. However, if, in addition to the table height, another item (e.g., beam width) has a different set value, a beam width difference ΔW is preferably calculated in addition to the table height difference ΔH and the three-dimensional scattered radiation distribution A30 is preferably corrected based on ΔH and ΔW.

Note that in FIG. 27, a case in which the start angle identified in step ST82 is "30°" is described. Next, as another example, a case in which the start angle identified in step ST82 is "160°" will be described. In this case, the flow in FIG. 14 is described as follows.

First, in step ST81, the processor of the computer 216 confirms the scan condition B of the subject body 112. Furthermore, in step ST82, the processor of the computer 216 identifies the start angle of the X-ray tube 104 when performing the half scan. Herein, it is assumed that the start angle of the X-ray tube is "160°". After identifying the start angle of the X-ray tube 104, the flow proceeds to step ST83.

In step ST83, the processor of the computer 216 determines whether the scan conditions X1 to Xm include a scan condition that matches the scan condition B of the subject body. Herein, as described above, it is assumed that the scan conditions X1 to Xm do not include the scan condition X1 that matches the scan condition B of the subject body. In this case, the processor selects one scan condition from the scan conditions X1 to Xm. The processor selects, for example, the scan condition X1.

In step ST84, the processor of the computer 216 determines whether the start angles 0° to 330° includes a start angle that matches the start angle identified in step ST82. In step ST82, the start angle is identified as 160°. Therefore, it is determined that the start angle of 160° identified in step ST82 is not included in the start angle of 0° to 330°. In this case, the processor selects a start angle of 150° that is closest to the starting angle of 160° from the plurality of start angles 0° to 330°.

In step ST85, the processor of the computer 216 determines the three-dimensional scattered radiation distribution corresponding to the second half scan based on the results of the determinations and selections in steps ST83 and ST84. This determination is performed as follows.

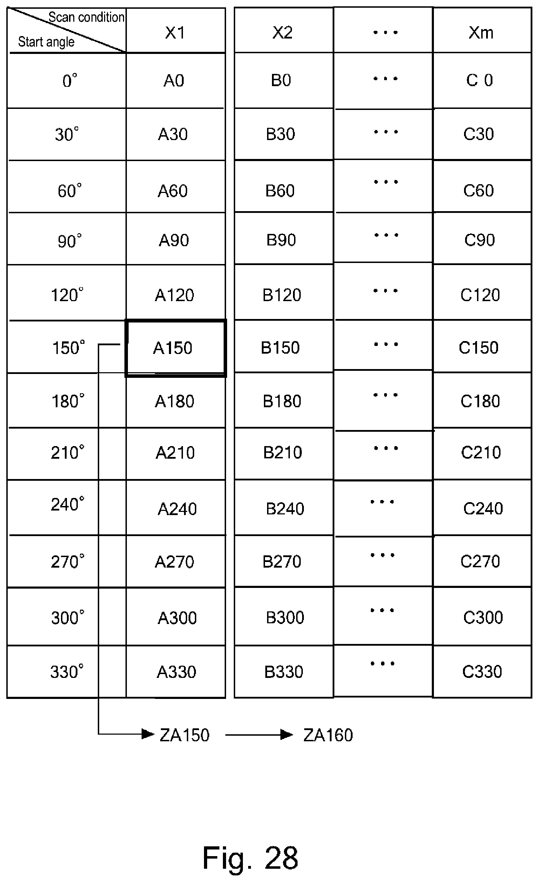

FIG. 28 is an explanatory diagram of step ST85.
The processor selects the three-dimensional scattered radiation distribution A150 corresponding to the scan condition X1 selected in step ST83 and the start angle of 150° selected in step ST84 from the n number of three-dimensional scattered radiation distributions A0 to C330. However, the scan condition X1 corresponding to the three-dimensional scattered radiation distribution A150 is different from the scan condition B of the subject body. Therefore, the processor compares the scan condition X1 with the scan condition B of the subject body and calculates the difference between the set values of each item in these two scan conditions. As described in FIG. 27, the heights of the table are different when comparing the scan condition X1 with the scan condition B of the subject body. Therefore, the processor calculates the difference ΔH (=H₃-H₁) in table heights. Furthermore, the processor corrects the three-dimensional scattered radiation distribution A150 based on the difference ΔH. In FIG. 28, the three-dimensional scattered radiation distribution obtained by correcting the three-dimensional scattered radiation distribution A150 is indicated by the code "ZA150".
Furthermore, the start angle of 150° corresponding to the three-dimensional scattered radiation distribution A150 is different from the start angle of 160° identified in step ST82. Therefore, the processor corrects the three-dimensional scattered radiation distribution ZA150 based on the angular difference between the start angle of 150° and the start angle of 160°. In FIG. 28, the three-dimensional scattered radiation distribution obtained by correcting the three-dimensional scattered radiation distribution ZA150 is indicated by the code "ZA160".

The three-dimensional scattered radiation distribution ZA160 obtained in this manner is determined as the three-dimensional scattered radiation distribution corresponding to the half scan, and the flow of step ST8 is terminated. Therefore, even if the scan conditions X1 to Xm do not include a scan condition that matches the scan condition B of the subject body, the three-dimensional scattered radiation distribution ZA160 corresponding to the scan condition B of the subject body can be obtained.

Note that FIG. 27 and FIG. 28 depict examples in which the scan condition X1 is selected. However, a different scan condition (e.g., scan condition Xm) may be selected than the scan condition X1. Furthermore, the similarity between each scan condition (X1 to Xm) and the scan condition B of the subject body 112 may be evaluated and the scan condition most similar to the scan condition B of the subject body 112 may be selected from the scan conditions X1 to Xm. By selecting the scan conditions based on similarity, a reliable three-dimensional scattered radiation distribution can be obtained.

### Embodiment 2

Embodiment 1 describes an example in which a half scan is performed at a point in time when the foot pedal is depressed. Embodiment 2 describes an example in which a half scan is performed to minimize the exposure of the surgeon to scattered radiation.

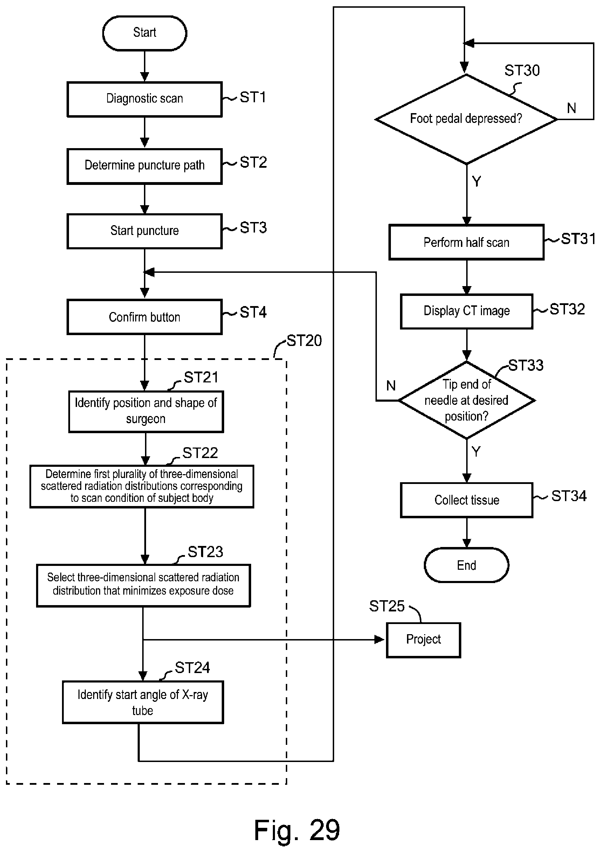

FIG. 29 is a diagram depicting an operation flow of a CT system in Embodiment 2. Steps ST1 to ST4 are the same as in Embodiment 1, and thus a description is omitted.

In step ST4, when the surgeon 101 presses the confirm button, a confirmation signal is output to the CT system 100 indicating that it is confirmed that the puncture site may be scanned.

When the CT system 100 receives the confirmation signal, the gantry motor controller 212 rotates the X-ray tube 104 and detector 108 in accordance with the instruction from the processor in the computer 216. When the confirm button is pressed, the flow proceeds to step ST20.

In step ST20, the processor of the computer 216 determines the angle of the X-ray tube (start angle) when starting to perform the half scan. A method for determining the start angle of the X-ray tube will be described below.

In step ST21, the processor of the computer 216 identifies the position and shape of the surgeon 101 in the scan room based on a camera image acquired by the camera 235 at the point in time when the confirm button is pressed. After identifying the position and shape of the surgeon 101, the flow proceeds to step ST22.

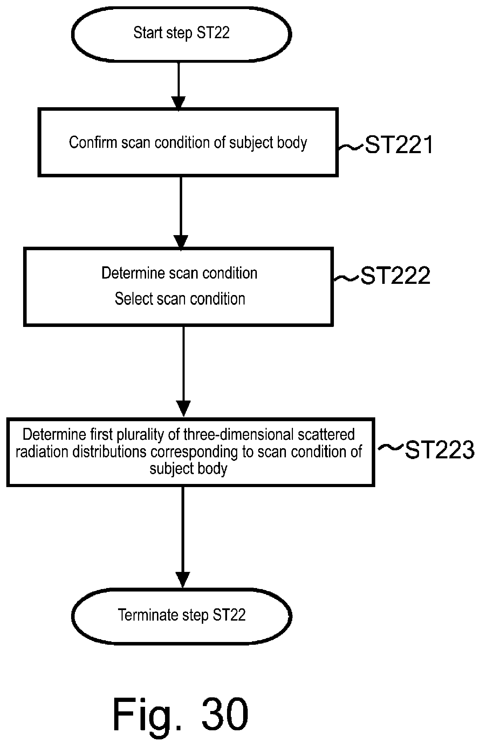

In step ST22, the processor of the computer 216 determines a plurality of three-dimensional scattered radiation distributions corresponding to the scan condition of the subject body 112. The determination method is described below while referring to FIG. 30.

FIG. 30 is a flowchart of step ST22.
In step ST221, the processor of the computer 216 confirms the scan condition B of the subject body 112. In Embodiment 1, the scan condition B for the subject body 112 is, for example, as follows.
Tube voltage: V₁ (kVp)
Beam width: W₁ (mm)
Size of subject body: Body height L₁ (cm), thickness T₁ (cm) of imaging site
Table height: H₁ (cm)

After confirming the scan condition B of the subject body 112, the flow proceeds to step ST222.
In step ST222, the processor of the computer 216 determines whether the scan conditions X1 to Xm (see FIG. 10) include a scan condition that matches the scan condition B of the subject body. Herein, it is assumed that the scan condition B of the subject body matches the scan condition X1. Therefore, the processor determines that scan condition X1 to Xm include the scan condition X1 that matches the scan condition B of the subject body. In this case, the processor selects the scan condition X1 that matches the scan condition B of the subject body from the scan conditions X1 to Xm.

In step ST223, the processor of the computer 216 selects the three-dimensional scattered radiation distributions A0 to A330 (scattered radiation distribution data set DS1) corresponding to the scan condition X1 from the n number of three-dimensional scattered radiation distributions A0 to C330 (see FIG. 10).
The three-dimensional scattered radiation distributions A0 to A330 selected in this manner can be determined as the plurality of three-dimensional scattered radiation distributions corresponding to the scan condition B for the subject body 112. After selecting the three-dimensional scattered radiation distributions A0 to A330, the flow depicted in FIG. 30 is terminated. Furthermore, the flow proceeds to step ST23 (see FIG. 29).

In step ST23, the processor of the computer 216 selects the three-dimensional scattered radiation distribution that minimizes the exposure of the surgeon 101 to scattered radiation from the three-dimensional scattered radiation distributions A0 to A330 selected in step ST22.

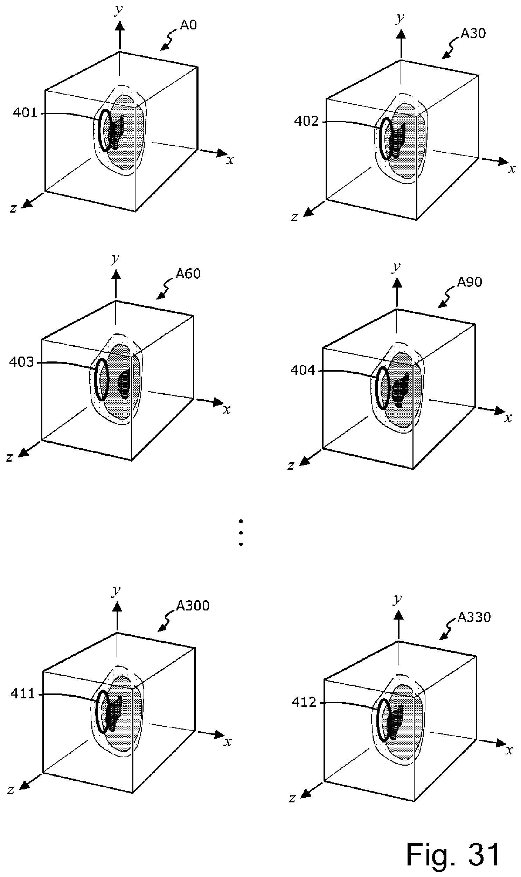

FIG. 31 is an explanatory diagram of a method for selecting a three-dimensional scattered radiation distribution that minimizes exposure of the surgeon 101 in step ST23.
First, the processor identifies the scattered radiation distribution in the region where the surgeon 101 is present for each three-dimensional scattered radiation distribution. FIG. 31 depicts a condition in which scattered radiation distributions 401 to 412 in the region where the surgeon 101 is present are identified with respect to the three-dimensional scattered radiation distributions A0 to A330, respectively. The processor compares the identified scattered radiation distributions 401 to 412 against each other for the three-dimensional scattered radiation distributions A0 to A330 and selects the three-dimensional scattered radiation distribution that minimizes the exposure of the surgeon 101 to scattered radiation from the three-dimensional scattered radiation distributions A0 to A330. Herein, it is assumed that the three-dimensional scattered radiation distribution A60 is the three-dimensional scattered radiation distribution that minimizes the exposure of the surgeon 101. Therefore, the processor selects the three-dimensional scattered radiation distribution A60 as the three-dimensional scattered radiation distribution that minimizes the exposure of the surgeon 101 from the three-dimensional scattered radiation distributions A0 to A330. After selecting the three-dimensional scattered radiation distribution A60, the flow proceeds to step ST24.

In step ST24, the processor of the computer 216 identifies the start angle of the X-ray tube 104 corresponding to the three-dimensional scattered radiation distribution A60 selected in step ST23. The three-dimensional scattered radiation distribution A60 corresponds to a start angle of 60°, and therefore, the processor identifies the start angle of the X-ray tube 104 corresponding to the three-dimensional scattered radiation distribution A60 as 60°.

In this manner, the start angle (60°) of the X-ray tube 104 can be determined. Once the start angle of the X-ray tube 104 is determined, the flow proceeds to step ST30.

In step ST30, the computer processor determines whether or not the foot pedal is depressed. If it is determined that the foot pedal is depressed, the flow proceeds to step ST31.
In step ST31, the first half scan is performed.

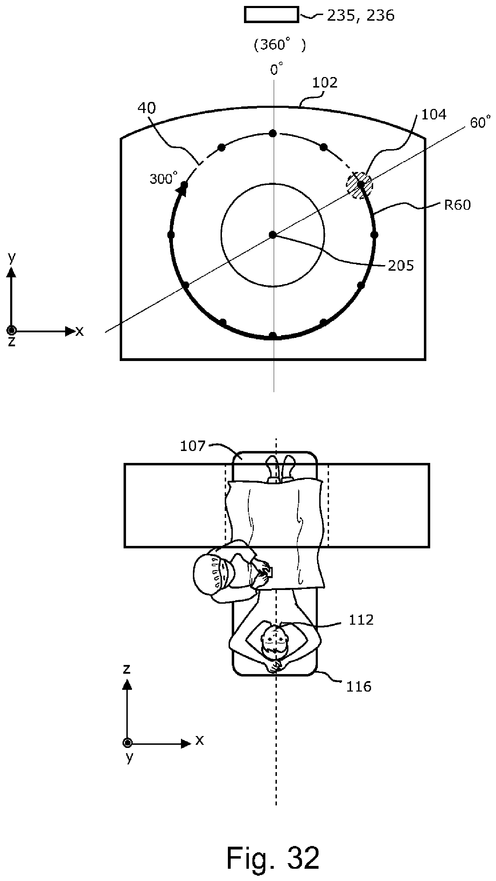

FIG. 32 is an explanatory diagram of the first half scan.
If it is determined that the foot pedal is depressed in step ST30, the processor of the computer 216 determines whether or not the X-ray tube 104 rotating on the path 40 has reached the start angle (60°) determined in step ST20. Furthermore, at the point in time when the X-ray tube 104 reaches the angle of 60°, the X-ray tube 104 begins irradiating X-rays.

Once X-ray irradiation begins, a half scan is performed until the X-ray tube 104 rotates from the start angle of 60° by 180° + 60° = 240°. Furthermore, when the X-ray tube 104 reaches the angle of 300°, the X-ray tube 104 finishes irradiating X-rays. In this manner, the half scan is completed.

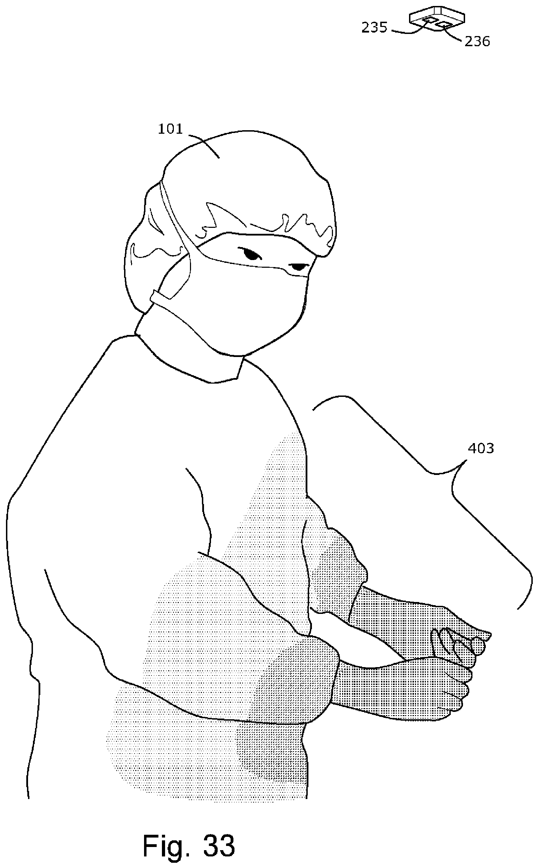

On the other hand, if the three-dimensional scattered radiation distribution A60 that minimizes exposure is selected in step ST23, step ST25 is performed. In step ST25, the processor of the computer 216 projects the scattered radiation distribution 403 (see FIG. 31) of the three-dimensional scattered radiation distribution A60 that minimizes exposure onto the surgeon 101 using the projection device 236, as depicted in FIG. 33. Therefore, the surgeon 101 can visually recognize the level of exposure to scattered radiation during the puncture.

Furthermore, if the half scan is performed in step ST31, the flow proceeds to step ST32.
In step ST32, the processor of the computer 216 reconstructs a CT image of a puncture portion based on data obtained by the half scan. Furthermore, the processor displays the reconstructed CT image on an interventional monitor 237.

In step ST33, the surgeon 101 confirms the position of the needle 301 while viewing a CT image displayed on the intervention monitor 237 to determine if the tip end of the needle 301 has reached a desired position. If the needle 301 has reached the desired position, the flow proceeds to step ST34, tissue is collected, and then the flow ends. On the other hand, if the tip end of the needle 301 has not reached the desired position, the flow returns to step ST4. Herein, it is determined that the tip end of the needle 301 has not yet reached the desired position. Therefore, the flow returns to step ST4.

In the same manner, steps ST4 to ST33 are repeated until the tip end of the needle 301 is determined to be at the desired position in step ST33. Therefore, each time a half scan is performed, the scattered radiation distribution (see FIG. 33) or cumulative scattered radiation distribution (see FIG. 25) is projected onto the surgeon 101 in step ST25. Thus, the surgeon 101 can visually recognize the level of exposure to scattered radiation. Furthermore, in step ST33, if the tip end of the needle is determined to be in the desired position, tissue is collected in step ST34 and the flow ends.

In Embodiment 2, a half scan is initiated at the point in time when the X-ray tube 104 reaches an angle that minimizes the exposure of the surgeon 101. Therefore, the exposure of the surgeon to scattered radiation can be minimized.

Note that the surgeon 101 may input a position signal representing the standing position of the surgeon using a user interface such as a touch panel or the like, and based on the position signal, select a three-dimensional scattered radiation distribution from a plurality of three-dimensional scattered radiation distributions A0 to A330. The position signal representing the standing position of the surgeon 101 can be, for example, a signal indicating whether the surgeon 101 is standing to the right or left of the table 116 when the table 116 is viewed from the gantry 102 side.

Note that Embodiment 2 describes a case in which it is determined that the scan conditions X1 to Xm include a scan condition that matches the scan condition B of the subject body 112 in step ST222 (see FIG. 30). However, depending on the set value of the scan condition B for the subject body 112, the scan conditions X1 to Xm may not include a scan condition that matches the scan condition B for the subject body 112. Therefore, the flow in FIG. 30 is described below, in which the scan conditions X1 to Xm do not include a scan condition that matches the scan condition B for the subject body 112.

In step ST221, the processor of the computer 216 confirms the scan condition B of the subject body 112. After confirming the scan condition B, the flow proceeds to step ST222.

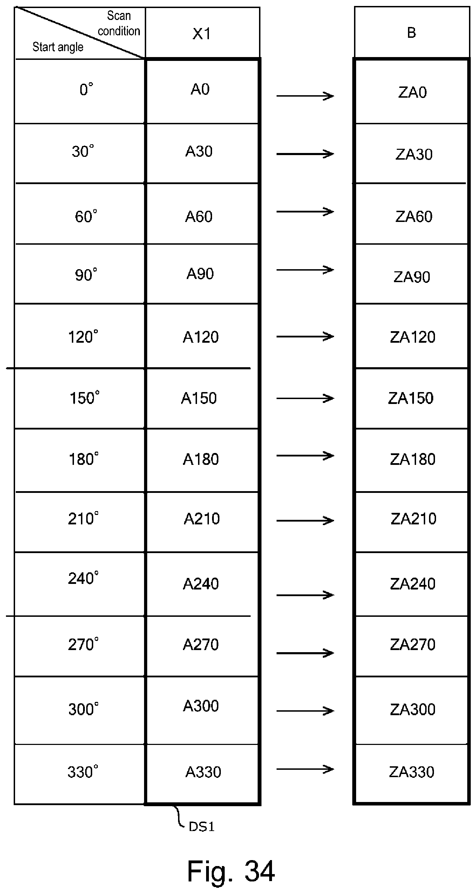

FIG. 34 is an explanatory diagram of step ST222 and step ST223.
In step ST222, the processor of the computer 216 determines whether the scan conditions X1 to Xm include a scan condition that matches the scan condition B of the subject body. Herein, as described above, it is assumed that the scan conditions X1 to Xm do not include the scan condition X1 that matches the scan condition B of the subject body. In this case, the processor selects one scan condition from the scan conditions X1 to Xm. FIG. 34 depicts an example in which the scan condition X1 is selected from the scan conditions X1 to Xm. After selecting the scan condition X1, the three-dimensional scattered radiation distributions A0 to A330 corresponding to the selected the scan condition X1 are selected from the n number of three-dimensional scattered radiation distributions A0 to C330 (see FIG. 10). Furthermore, in step ST223, the three-dimensional scattered radiation distributions A0 to A330 corresponding to the scan condition X1 are corrected based on the difference between the set value of the selected scan condition X1 and the set value of the scan condition B for the subject body 112. Thereby, the three-dimensional scattered radiation distributions ZA0 to ZA300 corresponding to the scan condition B of the subject body 112 can be determined. For example, if the table height for the scan condition B of the subject body is H₃ and the table height for the scan condition X1 is H₁, the three-dimensional scattered radiation distributions A0 to A330 corresponding to the scan condition X1 can be corrected based on the table height difference ΔH (= H₃ - H₁) to obtain the three-dimensional scattered radiation distributions ZA0 to ZA300 corresponding to the scan condition B for the subject body 112. Furthermore, if, in addition to the table height, another item (e.g., beam width) is different, a beam width difference ΔW is calculated in addition to the table height difference ΔH and the three-dimensional scattered radiation distributions A0 to A330 corresponding to the scan condition X1 are corrected based on ΔH and ΔW, such that the three-dimensional scattered radiation distributions ZA0 to ZA300 can be obtained. Therefore, even if the scan conditions X1 to Xm do not include a scan condition that matches the scan condition B of the subject body, the three-dimensional scattered radiation distributions ZA0 to ZA300 corresponding to the scan condition B of the subject body can be obtained.

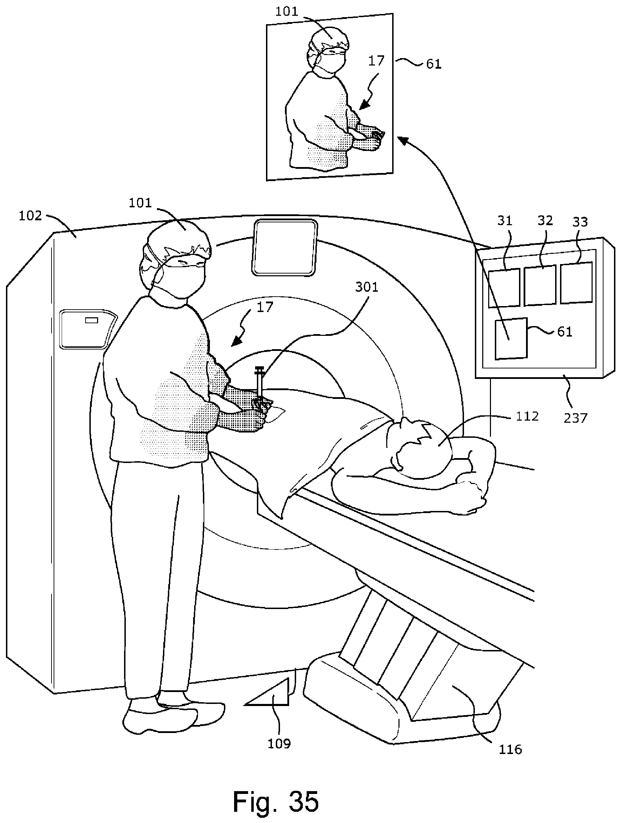

Note that Embodiments 1 and 2 depict examples of displaying the scattered radiation distribution by using a projection device to project the scattered radiation distribution onto the surgeon. However, the present invention is not limited to the example of using a projection device so long as the scattered radiation distribution can be displayed. The processor may, for example, display the scattered radiation distribution using a display device (see FIG. 35).

FIG. 35 is a diagram depicting an example in which a display device displays a scattered radiation distribution.
FIG. 35 depicts the interventional monitor 237 as an example of the display device.
An image 61 is displayed on the interventional monitor 237. An enlarged view of the image 61 is depicted on the upper side of FIG. 35. The image 61 includes the surgeon 101 and the scattered radiation distribution or cumulative scattered radiation distribution 17 superimposed on the body surface of the surgeon 101. By viewing the image 61, the surgeon 101 can visually recognize the level of exposure to scattered radiation when a single half scan is performed, or the cumulative exposure to scattered radiation when the half scan is repeatedly performed.

Note that although Embodiments 1 and 2 describe examples of performing a half scan, the present invention can also be applied when performing a full scan. For example, when a full scan is performed, as in ODM (Organ Dose Modulation), in which the dose of X-rays irradiated from the X-ray tube is reduced from a first dose to a second dose during one rotation of the X-ray tube, the three-dimensional scattered radiation distribution changes according to the start angle of the X-ray tube when the full scan is started. Therefore, even when a full scan is performed on the subject body, by preparing a three-dimensional scattered radiation distribution for each start angle of the X-ray tube of the subject body, the scattered radiation distribution in the region where the surgeon is present can be identified from the three-dimensional scattered radiation distribution, and the identified scattered radiation distribution can be projected onto the surgeon 101. This allows the surgeon 101 to visually recognize the degree of exposure.

Furthermore, the present invention can also be applied to a normal full scan that does not modulate the X-ray dose. In a normal full scan, the X-ray tube makes one rotation without modulation of the X-ray dose, and thus, the three-dimensional scattered radiation distribution is the same even if the start angle of the X-ray tube is changed. Therefore, by storing one three-dimensional scattered radiation distribution for each of the scan conditions X1 to Xm in the storage device, the scattered radiation distribution or cumulative scattered radiation distribution corresponding to the scan can be projected onto the surgeon or displayed on the display device.

Embodiments 1 and 2 describe cases of projecting the scattered radiation distribution or cumulative scattered radiation distribution onto the surgeon. However, the scattered radiation distribution that the subject body 112 receives from the scan may be identified, and the identified scattered radiation distribution may be projected onto the subject body 112, or the cumulative scattered radiation distribution may be projected onto the subject body 112. By projecting the scattered radiation distribution or cumulative scattered radiation distribution onto the subject body 112, not only the subject body itself but also the surgeon 101 can be aware of the exposure received by the subject body 112 from the scattered radiation. Therefore, the surgeon 101 can be made aware such that the subject body 112 receives the lowest possible exposure. Furthermore, the scattered radiation distribution or cumulative scattered radiation distribution of the subject body may be displayed on the display device.

Note that Embodiments 1 and 2 depicts examples of using a CT system as a medical system. However, the present invention is not limited to a CT system and can be applied to a system other than a CT system (e.g., IVR-CT system) as long as it is a medical system that irradiates an X-ray source onto a subject body.

### [REFERENCE SIGNS LIST]

11, 12, 250, 401 to 412. Scattered radiation distribution
13, 14. Cumulative scattered radiation distribution
17. Scattered radiation distribution or cumulative scattered radiation distribution
31, 32, 33. CT image
40, 140. Path
61. Image
100. CT system
101. Surgeon
102, 1102. Gantry
103. Filter part
104, 1104. X-ray tube
105. Front collimator
106. X-ray
107. Opening
108. X-ray detector
109. Foot pedal
112. Subject body
116, 1116. Table
118. Table motor controller
122. Scan room
124. Ceiling
150. Plane
202. Detector element
205, 1205. Rotation axis
210. X-ray controller
212. Gantry motor controller
214. DAS
216. Computer
218. Storage device
220. Operator console
224. PACS
230. image reconstructor
232. Display device
235. Camera
236. Projection device
237. Intervention monitor
300. Dosimeter
301. Needle

## Claims

1. A medical system, comprising:
a gantry (102) including an X-ray tube (104);
a table (116); and
one or more processors,
the one or more processors being arranged to execute operations including:
identifying the position and shape of a surgeon (101) and/or subject body (112) in a scan room (122) where the gantry (102) and table (116) are installed;
identifying, based on the position and shape of the surgeon (101) and/or subject body (112), a first scattered radiation distribution in a region of the scan room (122) in which the surgeon (101) and/or subject body (122) is present from a three-dimensional scattered radiation distribution that three-dimensionally expresses the distribution of scattered radiation generated by scanning the subject body (122); and
displaying the first scattered radiation distribution, wherein the X-ray tube (104) is arranged to rotate on a path along the circumference of a circle centered at a prescribed point, and
the three-dimensional scattered radiation distribution expresses the distribution of scattered radiations generated by the X-ray tube (104) irradiating X-rays as the X-ray tube (104) rotates between a start angle, which represents the angle of the X-ray tube (104) at the beginning of a scan, and an end angle, which represents the angle of the X-ray tube (104) at the end of the scan, wherein the one or more processors can communicate with a storage device,
the storage device stores an n number of three-dimensional scattered radiation distributions measured by changing a scan condition and the start angle of the X-ray tube (104), and
the n number of three-dimensional scattered radiation distributions are divided into a plurality of scattered radiation distribution data sets corresponding to a plurality of the scan conditions, and each scattered radiation distribution data set contains a plurality of three-dimensional scattered radiation distributions corresponding to a plurality of the start angles.

2. The medical system according to claim 1, wherein the scan condition includes at least one of the following items: tube voltage, beam width, patient size, and table height.

3. The medical system according to claim 1, wherein the one or more processors is arranged to determine the three-dimensional scattered radiation distribution corresponding to a scan performed on the subject body (112), and
identifying the first scattered radiation distribution includes identifying a first scattered radiation distribution from the determined three-dimensional scattered radiation distribution.

4. The medical system according to claim 3, wherein determining the three-dimensional scattered radiation distribution corresponding to the scan performed on the subject body (112) includes:
identifying the angle of the X-ray tube (104) at a time point at which a trigger for starting the scan occurs as the start angle of the X-ray tube (104);
if the plurality of scan conditions includes a first scan condition that matches the scan condition of the subject body (112), selecting the first scan condition from the plurality of scan conditions;
if the plurality of start angles includes a first start angle that matches the start angle of the identified X-ray tube (104), selecting the first start angle from the plurality of start angles; and
selecting a first three-dimensional scattered radiation distribution corresponding to the first scan condition and the first start angle from the n number of three-dimensional scattered radiation distributions, and
the selected first three-dimensional scattered radiation distribution is determined as the three-dimensional scattered radiation distribution corresponding to the scan performed on the subject body (112).

5. The medical system according to claim 3, wherein determining the three-dimensional scattered radiation distribution corresponding to the scan performed on the subject body (112) includes:
identifying the angle of the X-ray tube (104) at a time point at which a trigger for starting the scan occurs as the start angle of the X-ray tube (104);
if the plurality of scan conditions includes a first scan condition that matches the scan condition of the subject body, selecting the first scan condition from the plurality of scans;
if the plurality of start angles does not include a first start angle that matches the start angle of the identified X-ray tube (104), selecting a second start angle that is closest to the identified start angle from the plurality of start angles;
selecting a first three-dimensional scattered radiation distribution corresponding to the first scan condition and the second start angle from the n number of three-dimensional scattered radiation distributions; and
correcting the first three-dimensional scattered radiation distribution based on the angular difference between the second start angle and the identified start angle, and
the corrected first three-dimensional scattered radiation distribution is determined as the three-dimensional scattered radiation distribution corresponding to the scan performed on the subject body.

6. The medical system according to claim 3, wherein determining the three-dimensional scattered radiation distribution corresponding to the scan performed on the subject body (112) includes:
identifying the angle of the X-ray tube (104) at a time point at which a trigger for starting the scan occurs as the start angle of the X-ray tube (104);
if the plurality of scan conditions does not include a first scan condition that matches the scan condition of the subject body, selecting one scan condition from the plurality of scan conditions;
if the plurality of start angles includes a first start angle that matches the start angle of the identified X-ray tube (104), selecting the first start angle from the plurality of start angles;
selecting a first three-dimensional scattered radiation distribution corresponding to the one scan condition and the first start angle from the n number of three-dimensional scattered radiation distributions; and
correcting the first three-dimensional scattered radiation distribution based on the difference between the set value of each item in the selected one scan condition and the set value of each item in the scan condition of the subject body, and
the corrected first three-dimensional scattered radiation distribution is determined as the three-dimensional scattered radiation distribution corresponding to the scan performed on the subject body (112).

7. The medical system according to claim 3, wherein determining the three-dimensional scattered radiation distribution corresponding to the scan performed on the subject body (112) includes:
identifying the angle of the X-ray tube (104) at a time point at which a trigger for starting the scan occurs as the start angle of the X-ray tube (104);
if the plurality of scan conditions does not include a first scan condition that matches the scan condition of the subject body, selecting one scan condition from the plurality of scan conditions;
if the plurality of start angles does not include a first start angle that matches the start angle of the identified X-ray tube (104), selecting a second start angle that is closest to the identified start angle from the plurality of start angles;
selecting a first three-dimensional scattered radiation distribution corresponding to the one scan condition and the second start angle from the n number of three-dimensional scattered radiation distributions;
correcting the first three-dimensional scattered radiation distribution based on the difference between the set value of each item in the selected one scan condition and the set value of each item in the scan condition of the subject body as well as the angular difference between the second start angle and the identified start angle, and
the corrected first three-dimensional scattered radiation distribution is determined as the three-dimensional scattered radiation distribution corresponding to the scan performed on the subject body.

8. The medical system according to claim 1, wherein the one or more processors is arranged to:
determine a first plurality of three-dimensional scattered radiation distributions corresponding to a scan condition of the subject body,
select a three-dimensional scattered radiation distribution that minimizes the exposure of the surgeon (101) and/or subject body (112) to scattered radiation from the first plurality of three-dimensional scattered radiation distributions, and
identify the start angle of the X-ray tube (104) corresponding to the three-dimensional scattered radiation distribution that minimizes exposure, and
the X-ray tube (104) is arranged to start irradiation of X-rays at a time point when the identified start angle is reached.

9. The medical system according to claim 8, wherein identifying the first scattered radiation distribution includes identifying the first scattered radiation distribution from the three-dimensional scattered radiation distribution that minimizes the exposure.

10. The medical system according to claim 8, wherein determining the first plurality of three-dimensional scattered radiation distributions includes:
if the plurality of scan conditions includes a second scan condition that matches the scan condition of the subject body, selecting the second scan condition from the plurality of scan conditions; and
selecting a plurality of three-dimensional scattered radiation distributions corresponding to the second scan condition from the n number of three-dimensional scattered radiation distributions.

11. The medical system according to claim 8, wherein determining the first plurality of three-dimensional scattered radiation distributions includes:
if the plurality of scan conditions does not include a second scan condition that matches the scan condition of the subject body, selecting one scan condition from the plurality of scan conditions;
selecting a plurality of three-dimensional scattered radiation distributions corresponding to the selected one scan condition from the n number of three-dimensional scattered radiation distributions; and
correcting the selected plurality of three-dimensional scattered radiation distributions based on the difference between the set value of each item in the selected one scan condition and the set value of each item in the scan condition of the subject body.

12. The medical system according to claim 1, further comprising:
a user interface for inputting a position signal representing the standing position of the surgeon, the position signal being a signal indicating whether the surgeon is standing to on a right side or left side of the table (116) when the table (116) is viewed from the gantry (102) side, wherein
the one or more processors is arranged to select a three-dimensional scattered radiation distribution from the n number of three-dimensional scattered radiation distributions based on the position signal.

13. The medical system according to claim 1, wherein the one or more processors is arranged to display a cumulative scattered radiation distribution expressing the cumulative exposure to the surgeon or subject body by repeatedly scanning the subject body (112).

14. The medical system according to claim 1, wherein the scan performed on the subject body (112) is
a half scan, or
a full scan in which the dose of X-rays irradiated from the X-ray tube (104) is reduced from a first dose to a second dose during one rotation of the X-ray tube (104).

15. The medical system according to claim 1, wherein the first scattered radiation distribution is a scattered radiation distribution whose color hue, saturation, and/or brightness varies according to the intensity of the scattered radiation.

16. The medical system according to claim 1, wherein the one or more processors is arranged to:
project the scattered radiation distribution or cumulative scattered radiation distribution onto the surgeon (101) using a projection device, or
display an image containing the scattered radiation distribution or cumulative scattered radiation distribution superimposed on the surgeon and the body surface of the surgeon using a display device.

17. The medical system according to claim 1, wherein the one or more processors can communicate with an optical image acquisition unit for acquiring an optical image in the scan room (122), and
identifying the position and shape of the surgeon and/or subject body includes identifying the position and shape of the surgeon and/or the subject body based on the optical image.

## Patentansprüche

1. Medizinisches System, umfassend:
eine Gantry (102), die eine Röntgenröhre (104) aufweist;
einen Tisch (116); und
einen oder mehrere Prozessoren,
wobei die ein oder mehreren Prozessoren dazu ausgelegt sind, Operationen auszuführen, die Folgendes beinhalten:
Identifizieren der Position und Form eines Chirurgen (101) und/oder eines Subjektkörpers (112) in einem Scanraum (122), in dem die Gantry (102) und der Tisch (116) installiert sind;
Identifizieren, basierend auf der Position und Form des Chirurgen (101) und/oder des Subjektkörpers (112), einer ersten Streustrahlungsverteilung in einer Region des Scanraums (122), in dem sich der Chirurg (101) und/oder der Subjektkörper (122) befinden, anhand der dreidimensionalen Streustrahlungsverteilung, welche die Verteilung von Streustrahlung, die durch Abtasten des Subjektkörpers (122) generiert wird, dreidimensional ausdrückt; und
Anzeigen der ersten Streustrahlungsverteilung, wobei die Röntgenröhre (104) dazu ausgelegt ist, sich auf einem Pfad entlang des Umfangs eines an einem vorgegebenen Punkt zentrierten Kreises zu drehen, und
die dreidimensionale Streustrahlungsverteilung die Verteilung von Streustrahlungen ausdrückt, die von der Röntgenröhre (104) generiert werden, welche Röntgenstrahlen ausstrahlt, wenn sich die Röntgenröhre (104) zwischen einem Startwinkel, der den Winkel der Röntgenröhre (104) zu Beginn einer Abtastung darstellt, und einem Endwinkel, der den Winkel der Röntgenröhre (104) zum Ende der Abtastung darstellt, dreht, wobei die ein oder mehreren Prozessoren mit einer Datenspeichervorrichtung kommunizieren können,
wobei die Datenspeichervorrichtung eine Anzahl n von dreidimensionalen Streustrahlungsverteilungen speichert, die durch Ändern einer Abtastbedingung und des Startwinkels der Röntgenröhre (104) gemessen werden, und die Anzahl n von dreidimensionalen Streustrahlungsverteilungen in eine Mehrzahl von Streustrahlungsverteilungsdatensätzen unterteilt wird, die einer Mehrzahl der Abtastbedingungen entsprechen, und jeder Streustrahlungsverteilungsdatensatz eine Mehrzahl von dreidimensionalen Streustrahlungsverteilungen enthält, die einer Mehrzahl der Startwinkel entsprechen.

2. Medizinisches System gemäß Anspruch 1, wobei die Abtastbedingung wenigstens eine der folgenden Positionen beinhaltet: Röhrenspannung, Strahlbreite, Patientengröße und Tischhöhe.

3. Medizinisches System gemäß Anspruch 1, wobei die ein oder mehreren Prozessoren dazu ausgelegt sind, die dreidimensionale Streustrahlungsverteilung entsprechend einer Abtastung zu bestimmen, die an dem Subjektkörper (112) durchgeführt wird, und
das Identifizieren der ersten Streustrahlungsverteilung das Identifizieren einer ersten Streustrahlungsverteilung anhand der bestimmten dreidimensionalen Streustrahlungsverteilung beinhaltet.

4. Medizinisches System gemäß Anspruch 3, wobei das Bestimmen der dreidimensionalen Streustrahlungsverteilung entsprechend der Abtastung, die an dem Subjektkörper (112) durchgeführt wird, dies beinhaltet:
Identifizieren des Winkels der Röntgenröhre (104) zu einem Zeitpunkt, an dem ein Auslöser zum Starten der Abtastung als Startwinkel der Röntgenröhre (104) auftritt;
falls die Mehrzahl von Abtastbedingungen eine erste Abtastbedingung beinhaltet, die mit der Abtastbedingung des Subjektkörpers (112) übereinstimmt, Auswählen der ersten Abtastbedingung aus der Mehrzahl von Abtastbedingungen;
falls die Mehrzahl von Startwinkeln einen ersten Startwinkel beinhaltet, der mit dem Startwinkel der identifizierten Röntgenröhre (104) übereinstimmt,
Auswählen des ersten Startwinkels aus der Mehrzahl von Startwinkeln; und
Auswählen einer ersten dreidimensionalen Streustrahlungsverteilung, die der ersten Abtastbedingung und dem ersten Startwinkel entspricht, aus der Anzahl n von dreidimensionalen Streustrahlungsverteilungen und
wobei die ausgewählte dreidimensionale Streustrahlungsverteilung als die dreidimensionale Streustrahlungsverteilung bestimmt wird, die der Abtastung entspricht, welche an dem Subjektkörper (112) durchgeführt wird.

5. Medizinisches System gemäß Anspruch 3, wobei das Bestimmen der dreidimensionalen Streustrahlungsverteilung entsprechend der Abtastung, die an dem Subjektkörper (112) durchgeführt wird, dies beinhaltet:
Identifizieren des Winkels der Röntgenröhre (104) zu einem Zeitpunkt, an dem ein Auslöser zum Starten der Abtastung als Startwinkel der Röntgenröhre (104) auftritt;
falls die Mehrzahl von Abtastbedingungen eine erste Abtastbedingung beinhaltet, die mit der Abtastbedingung des Subjektkörpers übereinstimmt, Auswählen der ersten Abtastbedingung aus der der Mehrzahl von Abtastungen;
falls die Mehrzahl von Startwinkeln keinen ersten Startwinkel beinhaltet, der mit dem Startwinkel der identifizierten Röntgenröhre (104) übereinstimmt,
Auswählen eines zweiten Startwinkels, der dem identifizierten Startwinkel aus der Mehrzahl von Startwinkeln am nächsten kommt;
Auswählen einer ersten dreidimensionalen Streustrahlungsverteilung, die der ersten Abtastbedingung und dem zweiten Startwinkel entspricht, aus der Anzahl n von dreidimensionalen Streustrahlungsverteilungen; und
Korrigieren der ersten dreidimensionalen Streustrahlungsverteilung basierend auf der Winkeldifferenz zwischen dem zweiten Startwinkel und dem identifizierten Startwinkel und
wobei die korrigierte erste dreidimensionale Streustrahlungsverteilung als die dreidimensionale Streustrahlungsverteilung bestimmt wird, die der Abtastung entspricht, welche an dem Subjektkörper durchgeführt wird.

6. Medizinisches System gemäß Anspruch 3, wobei das Bestimmen der dreidimensionalen Streustrahlungsverteilung entsprechend der Abtastung, die an dem Subjektkörper (112) durchgeführt wird, dies beinhaltet:
Identifizieren des Winkels der Röntgenröhre (104) zu einem Zeitpunkt, an dem ein Auslöser zum Starten der Abtastung als Startwinkel der Röntgenröhre (104) auftritt;
falls die Mehrzahl von Abtastbedingungen keine erste Abtastbedingung beinhaltet, die mit der Abtastbedingung des Subjektkörpers übereinstimmt, Auswählen einer Abtastbedingung aus der Mehrzahl von Abtastbedingungen;
falls die Mehrzahl von Startwinkeln einen ersten Startwinkel beinhaltet, der mit dem Startwinkel der identifizierten Röntgenröhre (104) übereinstimmt, Auswählen des ersten Startwinkels aus der Mehrzahl von Startwinkeln;
Auswählen einer ersten dreidimensionalen Streustrahlungsverteilung, die der einen Abtastbedingung und dem ersten Startwinkel entspricht, aus der Anzahl n von dreidimensionalen Streustrahlungsverteilungen; und
Korrigieren der ersten dreidimensionalen Streustrahlungsverteilung basierend auf der Differenz zwischen dem eingestellten Wert jeder Position in der ausgewählten einen Abtastbedingung und dem eingestellten Wert jeder Position in der Abtastbedingung des Subjektkörpers und
wobei die korrigierte erste dreidimensionale Streustrahlungsverteilung als die dreidimensionale Streustrahlungsverteilung bestimmt wird, die der Abtastung entspricht, welche an dem Subjektkörper (112) durchgeführt wird.

7. Medizinisches System gemäß Anspruch 3, wobei das Bestimmen der dreidimensionalen Streustrahlungsverteilung entsprechend der Abtastung, die an dem Subjektkörper (112) durchgeführt wird, dies beinhaltet:
Identifizieren des Winkels der Röntgenröhre (104) zu einem Zeitpunkt, an dem ein Auslöser zum Starten der Abtastung als Startwinkel der Röntgenröhre (104) auftritt;
falls die Mehrzahl von Abtastbedingungen keine erste Abtastbedingung beinhaltet, die mit der Abtastbedingung des Subjektkörpers übereinstimmt, Auswählen einer Abtastbedingung aus der Mehrzahl von Abtastbedingungen;
falls die Mehrzahl von Startwinkeln keinen ersten Startwinkel beinhaltet, der mit dem Startwinkel der identifizierten Röntgenröhre (104) übereinstimmt, Auswählen eines zweiten Startwinkels, der dem identifizierten Startwinkel aus der Mehrzahl von Startwinkeln am nächsten kommt;
Auswählen einer ersten dreidimensionalen Streustrahlungsverteilung, die der einen Abtastbedingung und dem zweiten Startwinkel entspricht, aus der Anzahl n von dreidimensionalen Streustrahlungsverteilungen;
Korrigieren der ersten dreidimensionalen Streustrahlungsverteilung basierend auf der Differenz zwischen dem eingestellten Wert jeder Position in der ausgewählten einen Abtastbedingung und dem eingestellten Wert jeder Position in der Abtastbedingung des Subjektkörpers sowie der Winkeldifferenz zwischen dem zweiten Startwinkel und dem identifizierten Startwinkel und
wobei die korrigierte erste dreidimensionale Streustrahlungsverteilung als die dreidimensionale Streustrahlungsverteilung bestimmt wird, die der Abtastung entspricht, welche an dem Subjektkörper durchgeführt wird.

8. Medizinisches System gemäß Anspruch 1, wobei die ein oder mehreren Prozessoren hierzu ausgelegt sind:
Bestimmen einer ersten Mehrzahl von dreidimensionalen Streustrahlungsverteilungen entsprechend einer Abtastbedingung des Subjektkörpers,
Auswählen einer dreidimensionalen Streustrahlungsverteilung, welche die Exposition des Chirurgen (101) und/oder des Subjektkörpers (112) gegenüber Streustrahlung minimiert, aus der ersten Mehrzahl von dreidimensionalen Streustrahlungsverteilungen, und
Identifizieren des Startwinkels der Röntgenröhre (104) entsprechend der dreidimensionalen Streustrahlungsverteilung, die eine Exposition minimiert, und
wobei die Röntgenröhre (104) dazu ausgelegt ist, eine Ausstrahlung von Röntgenstrahlen zu einem Zeitpunkt zu starten, wenn der identifizierte Startwinkel erreicht ist.

9. Medizinisches System gemäß Anspruch 8, wobei das Identifizieren der ersten Streustrahlungsverteilung das Identifizieren der ersten Streustrahlungsverteilung anhand der dreidimensionalen Streustrahlungsverteilung beinhaltet, welche die Exposition minimiert.

10. Medizinisches System gemäß Anspruch gemäß Anspruch 8, wobei das Bestimmen der ersten Mehrzahl von dreidimensionalen Streustrahlungsverteilungen dies beinhaltet:
falls die Mehrzahl von Abtastbedingungen eine zweite Abtastbedingung beinhaltet, die mit der Abtastbedingung des Subjektkörpers übereinstimmt, Auswählen der zweiten Abtastbedingung aus der Mehrzahl von Abtastbedingungen; und
Auswählen einer Mehrzahl von dreidimensionalen Streustrahlungsverteilungen entsprechend der zweiten Abtastbedingung aus der Anzahl n von dreidimensionalen Streustrahlungsverteilungen.

11. Medizinisches System gemäß Anspruch gemäß Anspruch 8, wobei das Bestimmen der ersten Mehrzahl von dreidimensionalen Streustrahlungsverteilungen dies beinhaltet:
falls die Mehrzahl von Abtastbedingungen keine zweite Abtastbedingung beinhaltet, die mit der Abtastbedingung des Subjektkörpers übereinstimmt, Auswählen einer Abtastbedingung aus der Mehrzahl von Abtastbedingungen;
Auswählen einer Mehrzahl von dreidimensionalen Streustrahlungsverteilungen entsprechend der ausgewählten einen Abtastbedingung aus der Anzahl n von dreidimensionalen Streustrahlungsverteilungen; und
Korrigieren der ausgewählten Mehrzahl von dreidimensionalen Streustrahlungsverteilungen basierend auf der Differenz zwischen dem eingestellten Wert jeder Position in der ausgewählten einen Abtastbedingung und dem eingestellten Wert jeder Position in der Abtastbedingung des Subjektkörpers.

12. Medizinisches System gemäß Anspruch 1, ferner umfassend:
eine Benutzerschnittstelle zum Eingeben eines Positionssignals, welches die stehende Position des Chirurgen darstellt, wobei das Positionssignal ein Signal ist, das angibt, ob der Chirurg auf der rechten Seite oder der linken Seite des Tischs (116) steht, wenn der Tisch (116) von der Seite der Gantry (102) aus betrachtet wird, wobei
die ein oder mehreren Prozessoren dazu ausgelegt sind, eine dreidimensionale Streustrahlungsverteilung basierend auf dem Positionssignal aus der Anzahl n von dreidimensionalen Streustrahlungsverteilungen auszuwählen.

13. Medizinisches System gemäß Anspruch 1, wobei die ein oder mehreren Prozessoren dazu ausgelegt sind, eine kumulative Streustrahlungsverteilung anzuzeigen, welche die kumulative Exposition des Chirurgen oder des Subjektkörpers durch wiederholtes Abtasten des Subjektkörpers (112) ausdrückt.

14. Medizinisches System gemäß Anspruch 1, wobei die Abtastung, die an dem Subjektkörper (112) durchgeführt wird,
eine Halbabtastung ist oder
eine Vollabtastung ist, bei der die Dosis von Röntgenstrahlen, die von der Röntgenröhre (104) ausgestrahlt werden, während einer Drehung der Röntgenröhre (104) von einer ersten Dosis auf eine zweite Dosis reduziert wird.

15. Medizinisches System gemäß Anspruch 1, wobei die erste Streustrahlungsverteilung eine Streustrahlungsverteilung ist, deren Farbe, Farbton, Sättigung und/oder Helligkeit gemäß der Intensität der Streustrahlung variiert.

16. Medizinisches System gemäß Anspruch 1, wobei die ein oder mehreren Prozessoren hierzu ausgelegt sind:
Projizieren der Streustrahlungsverteilung oder der kumulativen Streustrahlungsverteilung auf den Chirurgen (101) unter Verwendung einer Projektionsvorrichtung oder Anzeigen eines Bildes, welches die Streustrahlungsverteilung oder die kumulative Streustrahlungsverteilung enthält, als Überlagerung auf den Chirurgen und die Körperoberfläche des Chirurgen unter Verwendung einer Anzeigevorrichtung.

17. Medizinisches System gemäß Anspruch 1, wobei die ein oder mehreren Prozessoren mit einer optischen Bilderfassungseinheit zum Erfassen eines optischen Bildes in dem Scanraum (122) kommunizieren können, und das Identifizieren der Position und Form des Chirurgen und/oder des Subjektkörpers das Identifizieren der Position und Form des Chirurgen und/oder des Subjektkörpers basierend auf dem optischen Bild beinhaltet.

## Revendications

1. Système médical, comprenant :
un portique (102) comportant un tube à rayons X (104) ;
une table (116) ; et
un ou plusieurs processeurs,
les un ou plusieurs processeurs étant conçus pour exécuter des opérations comportant :
l'identification de la position et de la forme d'un chirurgien (101) et/ou d'un corps de patient (112) dans une salle d'examen (122) où sont installés le portique (102) et la table (116) ;
l'identification, sur la base de la position et de la forme du chirurgien (101) et/ou du corps de patient (112), d'une première distribution de rayonnement diffusé dans une région de la salle d'examen (122) dans laquelle le chirurgien (101) et/ou le corps de patient (122) se trouvent à partir d'une distribution tridimensionnelle de rayonnement diffusé qui exprime tridimensionnellement la distribution d'un rayonnement diffusé généré par le balayage du corps de patient (122) ; et
l'affichage de la première distribution de rayonnement diffusé, dans lequel le tube à rayons X (104) est conçu pour tourner sur un trajet le long de la circonférence d'un cercle centré en un point prescrit, et
la distribution tridimensionnelle de rayonnement diffusé exprime la distribution de rayonnements diffusés générés par le tube à rayons X (104) diffusant des rayons X à mesure que le tube à rayons X (104) tourne entre un angle de départ, qui représente l'angle du tube à rayons X (104) au début d'un balayage, et un angle de fin, qui représente l'angle du tube à rayons X (104) à la fin du balayage, dans lequel les un ou plusieurs processeurs peuvent communiquer avec un dispositif de stockage,
le dispositif de stockage stocke un nombre n de distributions tridimensionnelles de rayonnement diffusé mesurées par modification d'une condition de balayage et de l'angle de départ du tube à rayons X (104), et
le nombre n de distributions tridimensionnelles de rayonnement diffusé est divisé en une pluralité d'ensembles de données de distributions de rayonnement diffusé correspondant à une pluralité de conditions de balayage, et chaque ensemble de données de distribution de rayonnement diffusé contient une pluralité de distributions tridimensionnelles de rayonnement diffusé correspondant à une pluralité d'angles de départ.

2. Système médical selon la revendication 1, dans lequel la condition de balayage comporte au moins l'un des éléments suivants : tension du tube, largeur du faisceau, taille du patient et hauteur de la table.

3. Système médical selon la revendication 1, dans lequel les un ou plusieurs processeurs sont conçus pour déterminer la distribution tridimensionnelle de rayonnement diffusé correspondant à un balayage effectué sur le corps de patient (112), et
l'identification de la première distribution de rayonnement diffusé consiste à identifier une première distribution de rayonnement diffusé à partir de la distribution tridimensionnelle de rayonnement diffusé déterminée.

4. Système médical selon la revendication 3, dans lequel la détermination de la distribution tridimensionnelle de rayonnement diffusé correspondant au balayage effectué sur le corps de patient (112) comporte :
l'identification, en tant qu'angle de départ du tube à rayons X (104), de l'angle du tube à rayons X (104) à un moment où se produit un déclenchement pour démarrer le balayage ;
si la pluralité de conditions de balayage comporte une première condition de balayage qui correspond à la condition de balayage du corps de patient (112), la sélection de la première condition de balayage dans la pluralité de conditions de balayage ;
si la pluralité d'angles de départ comporte un premier angle de départ qui correspond à l'angle de départ du tube à rayons X (104) identifié, la sélection du premier angle de départ dans la pluralité d'angles de départ ; et
la sélection d'une première distribution tridimensionnelle de rayonnement diffusé correspondant à la première condition de balayage et au premier angle de départ à partir du nombre n de distributions tridimensionnelles de rayonnement diffusé, et
la première distribution tridimensionnelle de rayonnement diffusé sélectionnée est déterminée en tant que distribution tridimensionnelle de rayonnement diffusé correspondant au balayage effectué sur le corps de patient (112).

5. Système médical selon la revendication 3, dans lequel la détermination de la distribution tridimensionnelle de rayonnement diffusé correspondant au balayage effectué sur le corps de patient (112) comporte :
l'identification, en tant qu'angle de départ du tube à rayons X (104), de l'angle du tube à rayons X (104) à un moment où se produit un déclenchement pour démarrer le balayage ;
si la pluralité de conditions de balayage comporte une première condition de balayage qui correspond à la condition de balayage du corps de patient, la sélection de la première condition de balayage dans la pluralité de balayages ;
si la pluralité d'angles de départ ne comporte pas un premier angle de départ qui correspond à l'angle de départ du tube à rayons X (104) identifié, la sélection d'un second angle de départ qui est le plus près de l'angle de départ identifié dans la pluralité d'angles de départ ;
la sélection d'une première distribution tridimensionnelle de rayonnement diffusé correspondant à la première condition de balayage et au second angle de départ à partir du nombre n de distributions tridimensionnelles de rayonnement diffusé ; et
la correction de la première distribution tridimensionnelle de rayonnement diffusé sur la base de la différence angulaire entre le second angle de départ et l'angle de départ identifié, et
la première distribution tridimensionnelle de rayonnement diffusé corrigée est déterminée en tant que distribution tridimensionnelle de rayonnement diffusé correspondant au balayage effectué sur le corps de patient.

6. Système médical selon la revendication 3, dans lequel la détermination de la distribution tridimensionnelle de rayonnement diffusé correspondant au balayage effectué sur le corps de patient (112) comporte :
l'identification, en tant qu'angle de départ du tube à rayons X (104), de l'angle du tube à rayons X (104) à un moment où se produit un déclenchement pour démarrer le balayage ;
si la pluralité de conditions de balayage ne comporte pas une première condition de balayage qui correspond à la condition de balayage du corps de patient, la sélection d'une condition de balayage dans la pluralité de conditions de balayage ;
si la pluralité d'angles de départ comporte un premier angle de départ qui correspond à l'angle de départ du tube à rayons X (104) identifié, la sélection du premier angle de départ dans la pluralité d'angles de départ ;
la sélection d'une première distribution tridimensionnelle de rayonnement diffusé correspondant à la condition de balayage et au premier angle de départ à partir du nombre n de distributions tridimensionnelles de rayonnement diffusé ; et
la correction de la première distribution de rayonnement diffusé tridimensionnel sur la base de la différence entre la valeur de consigne de chaque élément dans la condition de balayage sélectionnée et la valeur de consigne de chaque élément dans la condition de balayage du corps de patient, et
la première distribution tridimensionnelle de rayonnement diffusé corrigée est déterminée en tant que distribution tridimensionnelle de rayonnement diffusé correspondant au balayage effectué sur le corps de patient (112).

7. Système médical selon la revendication 3, dans lequel la détermination de la distribution tridimensionnelle de rayonnement diffusé correspondant au balayage effectué sur le corps de patient (112) comporte :
l'identification, en tant qu'angle de départ du tube à rayons X (104), de l'angle du tube à rayons X (104) à un moment où se produit un déclenchement pour démarrer le balayage ;
si la pluralité de conditions de balayage ne comporte pas une première condition de balayage qui correspond à la condition de balayage du corps de patient, la sélection d'une condition de balayage dans la pluralité de conditions de balayage ;
si la pluralité d'angles de départ ne comporte pas un premier angle de départ qui correspond à l'angle de départ du tube à rayons X (104) identifié, la sélection d'un second angle de départ qui est le plus près de l'angle de départ identifié dans la pluralité d'angles de départ ;
la sélection d'une première distribution tridimensionnelle de rayonnement diffusé correspondant à la condition de balayage et au second angle de départ à partir du nombre n de distributions tridimensionnelles de rayonnement diffusé ;
la correction de la première distribution tridimensionnelle de rayonnement diffusé sur la base de la différence entre la valeur de consigne de chaque élément dans la condition de balayage sélectionnée et la valeur de consigne de chaque élément dans la condition de balayage du corps de patient ainsi que de la différence angulaire entre le second angle de départ et l'angle de départ identifié, et
la première distribution tridimensionnelle de rayonnement diffusé corrigée est déterminée en tant que distribution tridimensionnelle de rayonnement diffusé correspondant au balayage effectué sur le corps de patient.

8. Système médical selon la revendication 1, dans lequel les un ou plusieurs processeurs sont conçus pour :
déterminer une première pluralité de distributions tridimensionnelles de rayonnement diffusé correspondant à une condition de balayage du corps de patient,
sélectionner une distribution tridimensionnelle de rayonnement diffusé qui réduit au minimum l'exposition du chirurgien (101) et/ou du corps de patient (112) au rayonnement diffusé à partir de la première pluralité de distributions tridimensionnelles de rayonnement diffusé, et
identifier l'angle de départ du tube à rayons X (104) correspondant à la distribution tridimensionnelle de rayonnement diffusé qui réduit l'exposition au minimum, et
le tube à rayons X (104) est conçu pour démarrer la diffusion de rayons X à un moment où l'angle de départ identifié est atteint.

9. Système médical selon la revendication 8, dans lequel l'identification de la première distribution de rayonnement diffusé comporte l'identification de la première distribution de rayonnement diffusé à partir de la distribution tridimensionnelle de rayonnement diffusé qui réduit l'exposition au minimum.

10. Système médical selon la revendication 8, dans lequel la détermination de la première pluralité de distributions tridimensionnelles de rayonnement diffusé comporte :
si la pluralité de conditions de balayage comporte une seconde condition de balayage qui correspond à la condition de balayage du corps de patient, la sélection de la seconde condition de balayage dans la pluralité de conditions de balayage ; et
la sélection d'une pluralité de distributions tridimensionnelles de rayonnement diffusé correspondant à la seconde condition de balayage à partir du nombre n de distributions tridimensionnelles de rayonnement diffusé.

11. Système médical selon la revendication 8, dans lequel la détermination de la première pluralité de distributions tridimensionnelles de rayonnement diffusé comporte :
si la pluralité de conditions de balayage ne comporte pas une seconde condition de balayage qui correspond à la condition de balayage du corps de patient, la sélection d'une condition de balayage dans la pluralité de conditions de balayage ;
la sélection d'une pluralité de distributions tridimensionnelles de rayonnement diffusé correspondant à la condition de balayage sélectionnée à partir du nombre n de distributions tridimensionnelles de rayonnement diffusé ; et
la correction de la pluralité sélectionnée de distributions tridimensionnelles de rayonnement diffusé sur la base de la différence entre la valeur de consigne de chaque élément dans la condition de balayage sélectionnée et la valeur de consigne de chaque élément dans la condition de balayage du corps de patient.

12. Système médical selon la revendication 1, comprenant en outre :
une interface utilisateur permettant d'entrer un signal de position représentant la position debout du chirurgien, le signal de position étant un signal indiquant si le chirurgien se tient debout sur un côté droit ou un côté gauche de la table (116) lorsque la table (116) est vue depuis le côté portique (102), dans lequel
les un ou plusieurs processeurs sont conçus pour sélectionner une distribution tridimensionnelle de rayonnement diffusé dans le nombre n de distributions tridimensionnelles de rayonnement diffusé sur la base du signal de position.

13. Système médical selon la revendication 1, dans lequel les un ou plusieurs processeurs sont conçus pour afficher une distribution cumulée de rayonnement diffusé exprimant l'exposition cumulée du chirurgien ou du corps de patient en balayant de manière répétée le corps de patient (112).

14. Système médical selon la revendication 1, dans lequel le balayage effectué sur le corps de patient (112) est
un demi-balayage, ou
un balayage complet dans lequel la dose de rayons X diffusés par le tube à rayons X (104) est réduite d'une première dose à une seconde dose pendant une rotation du tube à rayons X (104).

15. Système médical selon la revendication 1, dans lequel la première distribution de rayonnement diffusé est une distribution de rayonnement diffusé dont la teinte de couleur, la saturation et/ou la luminosité varient en fonction de l'intensité du rayonnement diffusé.

16. Système médical selon la revendication 1, dans lequel les un ou plusieurs processeurs sont conçus pour :
projeter la distribution de rayonnement diffusé ou la distribution cumulée de rayonnement diffusé sur le chirurgien (101) à l'aide d'un dispositif de projection, ou
afficher une image contenant la distribution de rayonnement diffusé ou la distribution cumulée de rayonnement diffusé superposée au chirurgien et à la surface corporelle du chirurgien à l'aide d'un dispositif d'affichage.

17. Système médical selon la revendication 1, dans lequel les un ou plusieurs processeurs peuvent communiquer avec une unité d'acquisition d'image optique pour acquérir une image optique dans la salle d'examen (122), et
l'identification de la position et de la forme du chirurgien et/ou du corps de patient comporte l'identification de la position et de la forme du chirurgien et/ou du corps de patient sur la base de l'image optique.
